# NEUE EUROPÄISCHE PATENTSCHRIFT

(11) **EP 1 257 290 B2**
(45) Veröffentlichungstag und Bekanntmachung des Hinweises auf die Entscheidung über den Einspruch: **27.03.2013**
(45) Hinweis auf die Patenterteilung: 07.01.2009
(21) Anmeldenummer: 01919295.4
(22) Anmeldetag: 17.02.2001
(51) Int. Cl.: A61K 39/00, A61K 39/245, A61K 38/08, A61K 38/10, A61K 38/16, G01N 33/68, G01N 33/53

(54) **VERFAHREN ZUR ANTIGEN-SPEZIFISCHEN STIMULATION VON T-LYMPHOZYTEN MIT SYNTHETISCHEN PEPTIDBIBLIOTHEKEN**
METHOD FOR ANTIGEN-SPECIFIC STIMULATION OF T-LYMPHOCYTES WITH SYNTHETIC PEPTIDE LIBRARIES
PROCEDE DE STIMULATION ANTIGENE SPECIFIQUE DE LYMPHOCYTES T AVEC DES BIBLIOTHEQUES PEPTIDIQUES DE SYNTHESE

(30) Priorität: 22.02.2000 DE 10009341
(43) Veröffentlichungstag der Anmeldung: 20.11.2002
(73) Patentinhaber: Kern, Florian, 10435 Berlin (DE)
(72) Erfinder: KERN, Florian, 10435 Berlin (DE); VOLK, Hans-Dieter, 10178 Berlin (DE); REINKE, Petra, 10178 Berlin (DE); FAULHABER, Nicole, 47509 Rheurdt (DE); SUREL, Ingolf-Pascal, 10315 Berlin (DE); KHATAMZAS, Elham, 10405 Berlin (DE)
(74) Vertreter: Schnappauf, Georg
(86) Internationale Anmeldenummer: PCT/EP2001/001773
(87) Internationale Veröffentlichungsnummer: WO 2001/063286

(56) Entgegenhaltungen:
- WO-A-99/36568
- KERN F ET AL: "Target structures of the CD8(+)-T-cell response to human cytomegalovirus: the 72-kilodalton major immediate-early protein revisited" CURRENT OPINION IN IMMUNOLOGY, CURRENT BIOLOGY LTD. LONDON, GB, Bd. 73, Nr. 10, Oktober 1999 (1999-10), Seiten 8179-8184, XP002125050 ISSN: 0952-7915 in der Anmeldung erwähnt
- F. KERN ET AL.: "T-cell epitope mapping by flow cytometry" NATURE MEDICINE, Bd. 4, Nr. 8, August 1998 (1998-08), Seiten 975-978, XP002179454 NEW YORK, N.Y., US
- F. KERN ET AL.: "Analysis of CD8 T cell reactivity to cytomegalovirus using protein-spanning pools of overlapping pentadecapeptides." EUROPEAN JOURNAL OF IMMUNOLOGY, Bd. 30, Nr. 6, Juni 2000 (2000-06), Seiten 1676-1682, XP001024972 WEINHEIM, DE in der Anmeldung erwähnt
- H.T. MAECKER ET AL.: "Use of overlapping peptide mixtures as antigens for cytokine flow cytometry." JOURNAL OF IMMUNOLOGICAL METHODS, Bd. 255, Nr. 1-2, 1. September 2001 (2001-09-01), Seiten 27-40, XP002179455 AMSTERDAM, NL
- HAMMER M ET AL.: "HLA type-independent generation of antigen-specific T cells for adoptive immunotherapy" EUR.J.IMMUNOL, Bd. 35, 2005, Seiten 2250-2258,
- JANEWAY CA AND TRAVENS P: "Immunologie" 1997, SPEKTRUM * Seite 31 - Seite 82 * * Seite 393 *
- VON BAEHR V: IMMUNODIAGNOSTIK, [Online] Seiten 1-9, [gefunden am 2005-07-20]

### SEQUENZPROTOKOLL

<110> Kern, Florian
<120> Verfahren zur antigen-spezifischen Stimulation von T-Lymphozyten mit synthetischen Peptidbibliotheken
<130> 010266wo/JH/ml
<140>
   <141>
<160> 260
<170> PatentIn Ver. 2.1
<210> 1
   <211> 491
   <212> PRT
   <213> Künstliche Sequenz
<220>
   <223> Beschreibung der künstlichen Sequenz: HCMV IE-1
<400> 1
   Met Glu Ser Ser Ala Lys Arg Lys Met Asp Pro Asp Asn Pro Asp Glu
   1 5 10 15
   Gly Pro Ser Ser Lys Val Pro Arg Pro Glu Thr Pro Val Thr Lys Ala
   20 25 30
   Thr Thr Phe Leu Gln Thr Met Leu Arg Lys Glu Val Asn Ser Gln Leu
   35 40 45
   Ser Leu Gly Asp Pro Leu Phe Pro Glu Leu Ala Glu Glu Ser Leu Lys
   50 55 60
   Thr Phe Glu Gln Val Thr Glu Asp Cys Asn Glu Asn Pro Glu Lys Asp
   65 70 75 80
   Val Leu Ala Glu Leu Val Lys Gln Ile Lys Val Arg Val Asp Met Val
   85 90 95
   Arg His Arg Ile Lys Glu His Met Leu Lys Lys Tyr Thr Gln Thr Glu
   100 105 110
   Glu Lys Phe Thr Gly Ala Phe Asn Met Met Gly Gly Cys Leu Gln Asn
   115 120 125
   Ala Leu Asp Ile Leu Asp Lys Val His Glu Pro Phe Glu Glu Met Lys
   130 135 140
   Cys Ile Gly Leu Thr Met Gln Ser Met Tyr Glu Asn Tyr Ile Val Pro
   145 150 155 160
   Glu Asp Lys Arg Glu Met Trp Met Ala Cys Ile Lys Glu Leu His Asp
   165 170 175
   Val Ser Lys Gly Ala Ala Asn Lys Leu Gly Gly Ala Leu Gln Ala Lys
   180 185 190
   Ala Arg Ala Lys Lys Asp Glu Leu Arg Arg Lys Met Met Tyr Met Cys
   195 200 205
   Tyr Arg Asn Ile Glu Phe Phe Thr Lys Asn Ser Ala Phe Pro Lys Thr
       210                 215                 220
   Thr Asn Gly Cys Ser Gln Ala Met Ala Ala Leu Gln Asn Leu Pro Gln
   225                 230                 235                 240
   Cys Ser Pro Asp Glu Ile Met Ala Tyr Ala Gln Lys Ile Phe Lys Ile
                   245                 250                 255
   Leu Asp Glu Glu Arg Asp Lys Val Leu Thr His Ile Asp His Ile Phe
               260                 265                 270
   Met Asp Ile Leu Thr Thr Cys Val Glu Thr Met Cys Asn Glu Tyr Lys
           275                 280                 285
   Val Thr Ser Asp Ala Cys Met Met Thr Met Tyr Gly Gly Ile Ser Leu
       290                 295                 300
   Leu Ser Glu Phe Cys Arg Val Leu Cys Cys Tyr Val Leu Glu Glu Thr
   305                 310                 315                 320
   Ser Val Met Leu Ala Lys Arg Pro Leu Ile Thr Lys Pro Glu Val Ile
                   325                 330                 335
   Ser Val Met Lys Arg Arg Ile Glu Glu Ile Cys Met Lys Val Phe Ala
               340                 345                 350
   Gln Tyr Ile Leu Gly Ala Asp Pro Leu Arg Val Cys Ser Pro Ser Val
           355                 360                 365
   Asp Asp Leu Arg Ala Ile Ala Glu Glu Ser Asp Glu Glu Glu Ala Ile
       370                 375                 380
   Val Ala Tyr Thr Leu Ala Thr Ala Gly Val Ser Ser Ser Asp Ser Leu
   385                 390                 395                 400
   Val Ser Pro Pro Glu Ser Pro Val Pro Ala Thr Ile Pro Leu Ser Ser
                   405                 410                 415
   Val Ile Val Ala Glu Asn Ser Asp Gln Glu Glu Ser Glu Gln Ser Asp
               420                 425                 430
   Glu Glu Glu Glu Glu Gly Ala Gln Glu Glu Arg Glu Asp Thr Val Ser
           435                 440                 445
   Val Lys Ser Glu Pro Val Ser Glu Ile Glu Glu Val Ala Pro Glu Glu
       450                 455                 460
   Glu Glu Asp Gly Ala Glu Glu Pro Thr Ala Ser Gly Gly Lys Ser Thr
   465                 470                 475                 480
   His Pro Met Val Thr Arg Ser Lys Ala Asp Gln
                   485                 490
<210> 2
   <211> 561
   <212> PRT
   <213> Künstliche Sequenz
<220>
   <223> Beschreibung der künstlichen Sequenz: HCMV pp65
<400> 2
   Met Glu Ser Arg Gly Arg Arg Cys Pro Glu Met Ile Ser Val Leu Gly
   1 5 10 15
   Pro Ile Ser Gly His Val Leu Lys Ala Val Phe Ser Arg Gly Asp Thr
   20 25 30
   Pro Val Leu Pro His Glu Thr Arg Leu Leu Gln Thr Gly Ile His Val
   35 40 45
   Arg Val Ser Gln Pro Ser Leu Ile Leu Val Ser Gln Tyr Thr Pro Asp
   50 55 60
   Ser Thr Pro Cys His Arg Gly Asp Asn Gln Leu Gln Val Gln His Thr
   65 70 75 80
   Tyr Phe Thr Gly Ser Glu Val Glu Asn Val Ser Val Asn Val His Asn
   85 90 95
   Pro Thr Gly Arg Ser Ile Cys Pro Ser Gln Glu Pro Met Ser Ile Tyr
   100 105 110
   Val Tyr Ala Leu Pro Leu Lys Met Leu Asn Ile Pro Ser Ile Asn Val
   115 120 125
   His His Tyr Pro Ser Ala Ala Glu Arg Lys His Arg His Leu Pro Val
   130 135 140
   Ala Asp Ala Val Ile His Ala Ser Gly Lys Gln Met Trp Gln Ala Arg
   145 150 155 160
   Leu Thr Val Ser Gly Leu Ala Trp Thr Arg Gln Gln Asn Gln Trp Lys
   165 170 175
   Glu Pro Asp Val Tyr Tyr Thr Ser Ala Phe Val Phe Pro Thr Lys Asp
   180 185 190
   Val Ala Leu Arg His Val Val Cys Ala His Glu Leu Val Cys Ser Met
   195 200 205
   Glu Asn Thr Arg Ala Thr Lys Met Gln Val Ile Gly Asp Gln Tyr Val
   210 215 220
   Lys Val Tyr Leu Glu Ser Phe Cys Glu Asp Val Pro Ser Gly Lys Leu
   225 230 235 240
   Phe Met His Val Thr Leu Gly Ser Asp Val Glu Glu Asp Leu Thr Met
   245 250 255
   Thr Arg Asn Pro Gln Pro Phe Met Arg Pro His Glu Arg Asn Gly Phe
   260 265 270
   Thr Val Leu Cys Pro Lys Asn Met Ile Ile Lys Pro Gly Lys Ile Ser
   275 280 285
   His Ile Met Leu Asp Val Ala Phe Thr Ser His Glu His Phe Gly Leu
   290 295 300
   Leu Cys Pro Lys Ser Ile Pro Gly Leu Ser Ile Ser Gly Asn Leu Leu
   305 310 315 320
   Met Asn Gly Gln Gln Ile Phe Leu Glu Val Gln Ala Ile Arg Glu Thr
   325 330 335
   Val Glu Leu Arg Gln Tyr Asp Pro Val Ala Ala Leu Phe Phe Phe Asp
   340 345 350
   Ile Asp Leu Leu Leu Gln Arg Gly Pro Gln Tyr Ser Glu His Pro Thr
   355 360 365
   Phe Thr Ser Gln Tyr Arg Ile Gln Gly Lys Leu Glu Tyr Arg His Thr
   370 375 380
   Trp Asp Arg His Asp Glu Gly Ala Ala Gln Gly Asp Asp Asp Val Trp
   385 390 395 400
   Thr Ser Gly Ser Asp Ser Asp Glu Glu Leu Val Thr Thr Glu Arg Lys
   405 410 415
   Thr Pro Arg Val Thr Gly Gly Gly Ala Met Ala Gly Ala Ser Thr Ser
   420 425 430
   Ala Gly Arg Lys Arg Lys Ser Ala Ser Ser Ala Thr Ala Cys Thr Ser
   435 440 445
   Gly Val Met Thr Arg Gly Arg Leu Lys Ala Glu Ser Thr Val Ala Pro
   450 455 460
   Glu Glu Asp Thr Asp Glu Asp Ser Asp Asn Glu Ile His Asn Pro Ala
   465 470 475 480
   Val Phe Thr Trp Pro Pro Trp Gln Ala Gly Ile Leu Ala Arg Asn Leu
   485 490 495
   Val Pro Met Val Ala Thr Val Gln Gly Gln Asn Leu Lys Tyr Gln Glu
   500 505 510
   Phe Phe Trp Asp Ala Asn Asp Ile Tyr Arg Ile Phe Ala Glu Leu Glu
   515 520 525
   Gly Val Trp Gln Pro Ala Ala Gln Pro Lys Arg Arg Arg His Arg Gln
   530 535 540
   Asp Ala Leu Pro Gly Pro Cys Ile Ala Ser Thr Pro Lys Lys His Arg
   545 550 555 560
   Gly
<210> 3
   <211> 15
   <212> PRT
   <213> Künstliche Sequenz
<220>
   <223> Beschreibung der künstlichen Sequenz: HCMV IE-1 Fragment
<400> 3
   Met Glu Ser Ser Ala Lys Arg Lys Met Asp Pro Asp Asn Pro Asp
   1 5 10 15
<210> 4
   <211> 15
   <212> PRT
   <213> Künstliche Sequenz
<220>
   <223> Beschreibung der künstlichen Sequenz: HCMV IE-1 Fragment
<40C> 4
<210> 5
   <211> 15
   <212> PRT
   <213> Künstliche Sequenz
<220>
   <223> Beschreibung der künstlichen Sequenz: HCMV IE-1 Fragment
<400> 5
   Ser Asp Ser Leu Val Ser Pro Pro Glu Ser Pro Val Pro Ala Thr
   1               5                   10                  15
<210> 6
   <211> 15
   <212> PRT
   <213> Künstliche Sequenz
<220>
   <223> Beschreibung der künstlichen Sequenz: HCMV IE-1 Fragment
<400> 6
   Val Ser Pro Pro Glu Ser Pro Val Pro Ala Thr Ile Pro Leu Ser
   1               5                   10                  15
<210> 7
   <211> 15
   <212> PRT
   <213> Künstliche Sequenz
<220>
   <223> Beschreibung der künstlichen Sequenz: HCMV IE-1 Fragment
<400> 7
   Glu Ser Pro Val Pro Ala Thr Ile Pro Leu Ser Ser Val Ile Val
   1               5                   10                  15
<210> 8
   <211> 15
   <212> PRT
   <213> Künstliche Sequenz
<220>
   <223> Beschreibung der künstlichen Sequenz: HCMV IE-1 Fragment
<400> 8
   Pro Ala Thr Ile Pro Leu Ser Ser Val Ile Val Ala Glu Asn Ser
   1 5 10 15
<210> 9
   <211> 15
   <212> PRT
   <213> Künstliche Sequenz
<220>
   <223> Beschreibung der künstlichen Sequenz: HCMV IE-1 Fragment
<400> 9
   Pro Leu Ser Ser Val Ile Val Ala Glu Asn Ser Asp Gln Glu Glu
   1 5 10 15
<210> 10
   <211> 15
   <212> PRT
   <213> Künstliche Sequenz
<220>
   <223> Beschreibung der künstlichen Sequenz: HCMV IE-1 Fragment
<400> 10
   Val Ile Val Ala Glu Asn Ser Asp Gln Glu Glu Ser Glu Gln Ser
   1 5 10 15
<210> 11
   <211> 15
   <212> PRT
   <213> Künstliche Sequenz
<220>
   <223> Beschreibung der künstlichen Sequenz: HCMV IE-1 Fragment
<400> 11
   Glu Asn Ser Asp Gln Glu Glu Ser Glu Gln Ser Asp Glu Glu Glu
   1 5 10 15
<210> 12
   <211> 15
   <212> PRT
   <213> Künstliche Sequenz
<220>
   <223> Beschreibung der künstlichen Sequenz: HCMV IE-1 Fragment
<400> 12
   Gln Glu Glu Ser Glu Gln Ser Asp Glu Glu Glu Glu Glu Gly Ala
    1               5                   10                  15
<210> 13
   <211> 15
   <212> PRT
   <213> Künstliche Sequenz
<220>
   <223> Beschreibung der künstlichen Sequenz: HCMV IE-1 Fragment
<400> 13
   Glu Gln Ser Asp Glu Glu Glu Glu Glu Gly Ala Gln Glu Glu Arg
    1               5                   10                  15
<210> 14
   <211> 15
   <212> PRT
   <213> Künstliche Sequenz
<220>
   <223> Beschreibung der künstlichen Sequenz: HCMV IE-1 Fragment
<400> 14
   Glu Glu Glu Glu Glu Gly Ala Gln Glu Glu Arg Glu Asp Thr Val
    1               5                   10                  15
<210> 15
   <211> 15
   <212> PRT
   <213> Künstliche Sequenz
<220>
   <223> Beschreibung der künstlichen Sequenz: HCMV IE-1 Fragment
<400> 15
   Arg Lys Glu Val Asn Ser Gln Leu Ser Leu Gly Asp Pro Leu Phe
    1               5                   10                  15
<210> 16
   <211> 15
   <212> PRT
   <213> Künstliche Sequenz
<220>
   <223> Beschreibung der künstlichen Sequenz: HCMV IE-1 Fragment
<400> 16
   Glu Gly Ala Gln Glu Glu Arg Glu Asp Thr Val Ser Val Lys Ser
   1               5                   10                  15
<210> 17
   <211> 15
   <212> PRT
   <213> Künstliche Sequenz
<220>
   <223> Beschreibung der künstlichen Sequenz: HCMV IE-1 Fragment
<400> 17
   Asn Ser Gln Leu Ser Leu Gly Asp Pro Leu Phe Pro Glu Leu Ala
   1               5                   10                  15
<210> 18
   <211> 15
   <212> PRT
   <213> Künstliche Sequenz
<220>
   <223> Beschreibung der künstlichen Sequenz: HCMV IE-1 Fragment
<400> 18
   Ser Leu Gly Asp Pro Leu Phe Pro Glu Leu Ala Glu Glu Ser Leu
   1               5                   10                  15
<210> 19
   <211> 15
   <212> PRT
   <213> Künstliche Sequenz
<220>
   <223> Beschreibung der künstlichen Sequenz: HCMV IE-1 Fragment
<400> 19
   Pro Leu Phe Pro Glu Leu Ala Glu Glu Ser Leu Lys Thr Phe Glu
   1 5 10 15
<210> 20
   <211> 15
   <212> PRT
   <213> Künstliche Sequenz
<220>
   <223> Beschreibung der künstlichen Sequenz: HCMV IE-1 Fragment
<400> 20
   Glu Leu Ala Glu Glu Ser Leu Lys Thr Phe Glu Gln Val Thr Glu
   1 5 10 15
<210> 21
   <211> 15
   <212> PRT
   <213> Künstliche Sequenz
<220>
   <223> Beschreibung der künstlichen Sequenz: HCMV IE-1 Fragment
<400> 21
   Glu Ser Leu Lys Thr Phe Glu Gln Val Thr Glu Asp Cys Asn Glu
   1 5 10 15
<210> 22
   <211> 15
   <212> PRT
   <213> Künstliche Sequenz
<220>
   <223> Beschreibung der künstlichen Sequenz: HCMV IE-1 Fragment
<400> 22
   Thr Phe Glu Gln Val Thr Glu Asp Cys Asn Glu Asn Pro Glu Lys
   1 5 10 15
<210> 23
   <211> 15
   <212> PRT
   <213> Künstliche Sequenz
<220>
   <223> Beschreibung der künstlichen Sequenz: HCMV IE-1 Fragment
<400> 23
   Val Thr Glu Asp Cys Asn Glu Asn Pro Glu Lys Asp Val Leu Ala
   1 5 10 15
<210> 24
   <211> 15
   <212> PRT
   <213> Künstliche Sequenz
<220>
   <223> Beschreibung der künstlichen Sequenz: HCMV IE-1 Fragment
<400> 24
   Cys Asn Glu Asn Pro Glu Lys Asp Val Leu Ala Glu Leu Val Lys
   1 5 10 15
<210> 25
   <211> 15
   <212> PRT
   <213> Künstliche Sequenz
<220>
   <223> Beschreibung der künstlichen Sequenz: HCMV IE-1 Fragment
<400> 25
   Ala Lys Arg Lys Met Asp Pro Asp Asn Pro Asp Glu Gly Pro Ser
   1 5 10 15
<210> 26
   <211> 15
   <212> PRT
   <213> Künstliche Sequenz
<220>
   <223> Beschreibung der künstlichen Sequenz: HCMV IE-1 Fragment
<400> 26
   Pro Glu Lys Asp Val Leu Ala Glu Leu Val Lys Gln Ile Lys Val
   1 5 10 15
<210> 27
   <211> 15
   <212> PRT
   <213> Künstliche Sequenz
<220>
   <223> Beschreibung der künstlichen Sequenz: HCMV IE-1 Fragment
<400> 27
   Val Leu Ala Glu Leu Val Lys Gln Ile Lys Val Arg Val Asp Met
   1               5                   10                  15
<210> 28
   <211> 15
   <212> PRT
   <213> Künstliche Sequenz
<220>
   <223> Beschreibung der künstlichen Sequenz: HCMV IE-1 Fragment
<400> 28
   Leu Val Lys Gln Ile Lys Val Arg Val Asp Met Val Arg His Arg
   1               5                   10                  15
<210> 29
   <211> 15
   <212> PRT
   <213> Künstliche Sequenz
<220>
   <223> Beschreibung der künstlichen Sequenz: HCMV IE-1 Fragment
<400> 29
   Ile Lys Val Arg Val Asp Met Val Arg His Arg Ile Lys Glu His
   1               5                   10                  15
<210> 30
   <211> 15
   <212> PRT
   <213> Künstliche Sequenz
<220>
   <223> Beschreibung der künstlichen Sequenz: HCMV IE-1 Fragment
<400> 30
   Val Asp Met Val Arg His Arg Ile Lys Glu His Met Leu Lys Lys
   1               5                   10                  15
<210> 31
   <211> 15
   <212> PRT
   <213> Künstliche Sequenz
<220>
   <223> Beschreibung der künstlichen Sequenz: HCMV IE-1 Fragment
<400> 31
   Arg His Arg Ile Lys Glu His Met Leu Lys Lys Tyr Thr Gln Thr
   1               5                   10                  15
<210> 32
   <211> 15
   <212> PRT
   <213> Künstliche Sequenz
<220>
   <223> Beschreibung der künstlichen Sequenz: HCMV IE-1 Fragment
<400> 32
   Lys Glu His Met Leu Lys Lys Tyr Thr Gln Thr Glu Glu Lys Phe
   1               5                   10                  15
<210> 33
   <211> 15
   <212> PRT
   <213> Künstliche Sequenz
<220>
   <223> Beschreibung der künstlichen Sequenz: HCMV IE-1 Fragment
<400> 33
   Leu Lys Lys Tyr Thr Gln Thr Glu Glu Lys Phe Thr Gly Ala Phe
   1               5                   10                  15
<210> 34
   <211> 15
   <212> PRT
   <213> Künstliche Sequenz
<220>
   <223> Beschreibung der künstlichen Sequenz: HCMV IE-1 Fragment
<400> 34
   Thr Gln Thr Glu Glu Lys Phe Thr Gly Ala Phe Asn Met Met Gly
   1               5                   10                  15
<210> 35
   <211> 15
   <212> PRT
   <213> Künstliche Sequenz
<220>
   <223> Beschreibung der künstlichen Sequenz: HCMV IE-1 Fragment
<400> 35
   Glu Lys Phe Thr Gly Ala Phe Asn Met Met Gly Gly Cys Leu Gln
   1 5 10 15
<210> 36
   <211> 15
   <212> PRT
   <213> Künstliche Sequenz
<220>
   <223> Beschreibung der künstlichen Sequenz: HCMV IE-1 Fragment
<400> 36
   Met Asp Pro Asp Asn Pro Asp Glu Gly Pro Ser Ser Lys Val Pro
   1 5 10 15
<210> 37
   <211> 15
   <212> PRT
   <213> Künstliche Sequenz
<220>
   <223> Beschreibung der künstlichen Sequenz: HCMV IE-1 Fragment
<400> 37
   Gly Ala Phe Asn Met Met Gly Gly Cys Leu Gln Asn Ala Leu Asp
   1 5 10 15
<210> 38
   <211> 15
   <212> PRT
   <213> Künstliche Sequenz
<220>
   <223> Beschreibung der künstlichen Sequenz: HCMV IE-1 Fragment
<400> 38
   Met Met Gly Gly Cys Leu Gln Asn Ala Leu Asp Ile Leu Asp Lys
   1 5 10 15
<210> 39
   <211> 15
   <212> PRT
   <213> Künstliche Sequenz
<220>
   <223> Beschreibung der künstlichen Sequenz: HCMV IE-1 Fragment
<400> 39
   Cys Leu Gln Asn Ala Leu Asp Ile Leu Asp Lys Val His Glu Pro
   1 5 10 15
<210> 40
   <211> 15
   <212> PRT
   <213> Künstliche Sequenz
<220>
   <223> Beschreibung der künstlichen Sequenz: HCMV IE-1 Fragment
<400> 40
   Ala Leu Asp Ile Leu Asp Lys Val His Glu Pro Phe Glu Glu Met
   1 5 10 15
<210> 41
   <211> 15
   <212> PRT
   <213> Künstliche Sequenz
<220>
   <223> Beschreibung der künstlichen Sequenz: HCMV IE-1 Fragment
<400> 41
   Leu Asp Lys Val His Glu Pro Phe Glu Glu Met Lys Cys Ile Gly
   1 5 10 15
<210> 42
   <211> 15
   <212> PRT
   <213> Künstliche Sequenz
<220>
   <223> Beschreibung der künstlichen Sequenz: HCMV IE-1 Fragment
<400> 42
   His Glu Pro Phe Glu Glu Met Lys Cys Ile Gly Leu Thr Met Gln
   1 5 10 15
<210> 43
   <211> 15
   <212> PRT
   <213> Künstliche Sequenz
<220>
   <223> Beschreibung der künstlichen Sequenz: HCMV IE-1 Fragment
<400> 43
   Glu Glu Met Lys Cys Ile Gly Leu Thr Met Gln Ser Met Tyr Glu
   1 5 10 15
<210> 44
   <211> 15
   <212> PRT
   <213> Künstliche Sequenz
<220>
   <223> Beschreibung der künstlichen Sequenz: HCMV IE-1 Fragment
<400> 44
   Cys Ile Gly Leu Thr Met Gln Ser Met Tyr Glu Asn Tyr Ile Val
   1 5 10 15
<210> 45
   <211> 15
   <212> PRT
   <213> Künstliche Sequenz
<220>
   <223> Beschreibung der künstlichen Sequenz: HCMV IE-1 Fragment
<400> 45
   Thr Met Gln Ser Met Tyr Glu Asn Tyr Ile Val Pro Glu Asp Lys
   1 5 10 15
<210> 46
   <211> 15
   <212> PRT
   <213> Künstliche Sequenz
<220>
   <223> Beschreibung der künstlichen Sequenz: HCMV IE-1 Fragment
<400> 46
   Met Tyr Glu Asn Tyr Ile Val Pro Glu Asp Lys Arg Glu Met Trp
   1 5 10 15
<210> 47
   <211> 15
   <212> PRT
   <213> Künstliche Sequenz
<220>
   <223> Beschreibung der künstlichen Sequenz: HCMV IE-1 Fragment
<400> 47
   Asn Pro Asp Glu Gly Pro Ser Ser Lys Val Pro Arg Pro Glu Thr
   1 5 10 15
<210> 48
   <211> 15
   <212> PRT
   <213> Künstliche Sequenz
<220>
   <223> Beschreibung der künstlichen Sequenz: HCMV IE-1 Fragment
<400> 48
   Tyr Ile Val Pro Glu Asp Lys Arg Glu Met Trp Met Ala Cys Ile
   1 5 10 15
<210> 49
   <211> 15
   <212> PRT
   <213> Künstliche Sequenz
<220>
   <223> Beschreibung der künstlichen Sequenz: HCMV IE-1 Fragment
<400> 49
   Glu Asp Lys Arg Glu Met Trp Met Ala Cys Ile Lys Glu Leu His
   1 5 10 15
<210> 50
   <211> 15
   <212> PRT
   <213> Künstliche Sequenz
<220>
   <223> Beschreibung der künstlichen Sequenz: HCMV IE-1 Fragment
<400> 50
   Glu Met Trp Met Ala Cys Ile Lys Glu Leu His Asp Val Ser Lys
   1 5 10 15
<210> 51
   <211> 15
   <212> PRT
   <213> Künstliche Sequenz
<220>
   <223> Beschreibung der künstlichen Sequenz: HCMV IE-1 Fragment
<400> 51
   Ala Cys Ile Lys Glu Leu His Asp Val Ser Lys Gly Ala Ala Asn
   1 5 10 15
<210> 52
   <211> 15
   <212> PRT
   <213> Künstliche Sequenz
<220>
   <223> Beschreibung der künstlichen Sequenz: HCMV IE-1 Fragment
<400> 52
   Glu Leu His Asp Val Ser Lys Gly Ala Ala Asn Lys Leu Gly Gly
   1 5 10 15
<210> 53
   <211> 15
   <212> PRT
   <213> Künstliche Sequenz
<220>
   <223> Beschreibung der künstlichen Sequenz: HCMV IE-1 Fragment
<400> 53
   Val Ser Lys Gly Ala Ala Asn Lys Leu Gly Gly Ala Leu Gln Ala
   1 5 10 15
<210> 54
   <211> 15
   <212> PRT
   <213> Künstliche Sequenz
<220>
   <223> Beschreibung der künstlichen Sequenz: HCMV IE-1 Fragment
<400> 54
   Ala Ala Asn Lys Leu Gly Gly Ala Leu Gln Ala Lys Ala Arg Ala
   1               5                   10                  15
<210> 55
   <211> 15
   <212> PRT
   <213> Künstliche Sequenz
<220>
   <223> Beschreibung der künstlichen Sequenz: HCMV IE-1 Fragment
<400> 55
   Leu Gly Gly Ala Leu Gln Ala Lys Ala Arg Ala Lys Lys Asp Glu
   1               5                   10                  15
<210> 56
   <211> 15
   <212> PRT
   <213> Künstliche Sequenz
<220>
   <223> Beschreibung der künstlichen Sequenz: HCMV IE-1 Fragment
<400> 56
   Leu Gln Ala Lys Ala Arg Ala Lys Lys Asp Glu Leu Arg Arg Lys
   1               5                   10                  15
<210> 57
   <211> 15
   <212> PRT
   <213> Künstliche Sequenz
<220>
   <223> Beschreibung der künstlichen Sequenz: HCMV IE-1 Fragment
<400> 57
   Ala Arg Ala Lys Lys Asp Glu Leu Arg Arg Lys Met Met Tyr Met
   1               5                   10                  15
<210> 58
   <211> 15
   <212> PRT
   <213> Künstliche Sequenz
<220>
   <223> Beschreibung der künstlichen Sequenz: HCMV IE-1 Fragment
<400> 58
   Gly Pro Ser Ser Lys Val Pro Arg Pro Glu Thr Pro Val Thr Lys
   1               5                   10                  15
<210> 59
   <211> 15
   <212> PRT
   <213> Künstliche Sequenz
<220>
   <223> Beschreibung der künstlichen Sequenz: HCMV IE-1 Fragment
<400> 59
   Lys Asp Glu Leu Arg Arg Lys Met Met Tyr Met Cys Tyr Arg Asn
   1               5                   10                  15
<210> 60
   <211> 15
   <212> PRT
   <213> Künstliche Sequenz
<220>
   <223> Beschreibung der künstlichen Sequenz: HCMV IE-1 Fragment
<400> 60
   Arg Arg Lys Met Met Tyr Met Cys Tyr Arg Asn Ile Glu Phe Phe
   1               5                   10                  15
<210> 61
   <211> 15
   <212> PRT
   <213> Künstliche Sequenz
<220>
   <223> Beschreibung der künstlichen Sequenz: HCMV IE-1 Fragment
<400> 61
   Met Tyr Met Cys Tyr Arg Asn Ile Glu Phe Phe Thr Lys Asn Ser
   1               5                   10                  15
<210> 62
   <211> 15
   <212> PRT
   <213> Künstliche Sequenz
<220>
   <223> Beschreibung der künstlichen Sequenz: HCMV IE-1 Fragment
<400> 62
   Tyr Arg Asn Ile Glu Phe Phe Thr Lys Asn Ser Ala Phe Pro Lys
   1               5                   10                  15
<210> 63
   <211> 15
   <212> PRT
   <213> Künstliche Sequenz
<220>
   <223> Beschreibung der künstlichen Sequenz: HCMV IE-1 Fragment
<400> 63
   Glu Phe Phe Thr Lys Asn Ser Ala Phe Pro Lys Thr Thr Asn Gly
   1               5                   10                  15
<210> 64
   <211> 15
   <212> PRT
   <213> Künstliche Sequenz
<220>
   <223> Beschreibung der künstlichen Sequenz: HCMV IE-1 fragments
<400> 64
   Lys Asn Ser Ala Phe Pro Lys Thr Thr Asn Gly Cys Ser Gln Ala
   1               5                   10                  15
<210> 65
   <211> 15
   <212> PRT
   <213> Künstliche Sequenz
<220>
   <223> Beschreibung der künstlichen Sequenz: HCMV IE-1 Fragment
<400> 65
   Phe Pro Lys Thr Thr Asn Gly Cys Ser Gln Ala Met Ala Ala Leu
   1               5                   10                  15
<210> 66
   <211> 15
   <212> PRT
   <213> Künstliche Sequenz
<220>
   <223> Beschreibung der künstlichen Sequenz: HCMV IE-1 Fragment
<400> 66
   Thr Asn Gly Cys Ser Gln Ala Met Ala Ala Leu Gln Asn Leu Pro
   1               5                   10                  15
<210> 67
   <211> 15
   <212> PRT
   <213> Künstliche Sequenz
<220>
   <223> Beschreibung der künstlichen Sequenz: HCMV IE-1 Fragment
<400> 67
   Ser Gln Ala Met Ala Ala Leu Gln Asn Leu Pro Gln Cys Ser Pro
   1               5                   10                  15
<210> 68
   <211> 15
   <212> PRT
   <213> Künstliche Sequenz
<220>
   <223> Beschreibung der künstlichen Sequenz: HCMV IE-1 Fragment
<400> 68
   Ala Ala Leu Gln Asn Leu Pro Gln Cys Ser Pro Asp Glu Ile Met
   1               5                   10                  15
<210> 69
   <211> 15
   <212> PRT
   <213> Künstliche Sequenz
<220>
   <223> Beschreibung der künstlichen Sequenz: HCMV IE-1 Fragment
<400> 69
   Lys Val Pro Arg Pro Glu Thr Pro Val Thr Lys Ala Thr Thr Phe
   1 5 10 15
<210> 70
   <211> 15
   <212> PRT
   <213> Künstliche Sequenz
<220>
   <223> Beschreibung der künstlichen Sequenz: HCMV IE-1 Fragment
<400> 70
   Asn Leu Pro Gln Cys Ser Pro Asp Glu Ile Met Ala Tyr Ala Gln
   1 5 10 15
<210> 71
   <211> 15
   <212> PRT
   <213> Künstliche Sequenz
<220>
   <223> Beschreibung der künstlichen Sequenz: HCMV IE-1 Fragment
<400> 71
   Cys Ser Pro Asp Glu Ile Met Ala Tyr Ala Gln Lys Ile Phe Lys
   1 5 10 15
<210> 72
   <211> 15
   <212> PRT
   <213> Künstliche Sequenz
<220>
   <223> Beschreibung der künstlichen Sequenz: HCMV IE-1 Fragment
<400> 72
   Glu Ile Met Ala Tyr Ala Gln Lys Ile Phe Lys Ile Leu Asp Glu
   1 5 10 15
<210> 73
   <211> 15
   <212> PRT
   <213> Künstliche Sequenz
<220>
   <223> Beschreibung der künstlichen Sequenz: HCMV IE-1 Fragment
<400> 73
   Tyr Ala Gln Lys Ile Phe Lys Ile Leu Asp Glu Glu Arg Asp Lys
   1 5 10 15
<210> 74
   <211> 15
   <212> PRT
   <213> Künstliche Sequenz
<220>
   <223> Beschreibung der künstlichen Sequenz: HCMV IE-1 Fragment
<400> 74
   Ile Phe Lys Ile Leu Asp Glu Glu Arg Asp Lys Val Leu Thr His
   1 5 10 15
<210> 75
   <211> 15
   <212> PRT
   <213> Künstliche Sequenz
<220>
   <223> Beschreibung der künstlichen Sequenz: HCMV IE-1 Fragment
<400> 75
   Leu Asp Glu Glu Arg Asp Lys Val Leu Thr His Ile Asp His Ile
   1 5 10 15
<210> 76
   <211> 15
   <212> PRT
   <213> Künstliche Sequenz
<220>
   <223> Beschreibung der künstlichen Sequenz: HCMV IE-1 Fragment
<400> 76
   Arg Asp Lys Val Leu Thr His Ile Asp His Ile Phe Met Asp Ile
   1 5 10 15
<210> 77
   <211> 15
   <212> PRT
   <213> Künstliche Sequenz
<220>
   <223> Beschreibung der künstlichen Sequenz: HCMV IE-1 Fragment
<400> 77
   Leu Thr His Ile Asp His Ile Phe Met Asp Ile Leu Thr Thr Cys
   1 5 10 15
<210> 78
   <211> 15
   <212> PRT
   <213> Künstliche Sequenz
<220>
   <223> Beschreibung der künstlichen Sequenz: HCMV IE-1 Fragment
<400> 78
   Asp His Ile Phe Met Asp Ile Leu Thr Thr Cys Val Glu Thr Met
   1 5 10 15
<210> 79
   <211> 15
   <212> PRT
   <213> Künstliche Sequenz
<220>
   <223> Beschreibung der künstlichen Sequenz: HCMV IE-1 Fragment
<400> 79
   Met Asp Ile Leu Thr Thr Cys Val Glu Thr Met Cys Asn Glu Tyr
   1 5 10 15
<210> 80
   <211> 15
   <212> PRT
   <213> Künstliche Sequenz
<220>
   <223> Beschreibung der künstlichen Sequenz: HCMV IE-1 Fragment
<400> 80
   Pro Glu Thr Pro Val Thr Lys Ala Thr Thr Phe Leu Gln Thr Met
   1 5 10 15
<210> 81
   <211> 15
   <212> PRT
   <213> Künstliche Sequenz
<220>
   <223> Beschreibung der künstlichen Sequenz: HCMV IE-1 Fragment
<400> 81
   Thr Thr Cys Val Glu Thr Met Cys Asn Glu Tyr Lys Val Thr Ser
   1 5 10 15
<210> 82
   <211> 15
   <212> PRT
   <213> Künstliche Sequenz
<220>
   <223> Beschreibung der künstlichen Sequenz: HCMV IE-1 Fragment
<400> 82
   Glu Thr Met Cys Asn Glu Tyr Lys Val Thr Ser Asp Ala Cys Met
   1 5 10 15
<210> 83
   <211> 15
   <212> PRT
   <213> Künstliche Sequenz
<220>
   <223> Beschreibung der künstlichen Sequenz: HCMV IE-1 Fragment
<400> 83
   Asn Glu Tyr Lys Val Thr Ser Asp Ala Cys Met Met Thr Met Tyr
   1 5 10 15
<210> 84
   <211> 15
   <212> PRT
   <213> Künstliche Sequenz
<220>
   <223> Beschreibung der künstlichen Sequenz: HCMV IE-1 Fragment
<400> 84
   Val Thr Ser Asp Ala Cys Met Met Thr Met Tyr Gly Gly Ile Ser
   1 5 10 15
<210> 85
   <211> 15
   <212> PRT
   <213> Künstliche Sequenz
<220>
   <223> Beschreibung der künstlichen Sequenz: HCMV IE-1 Fragment
<400> 85
   Ala Cys Met Met Thr Met Tyr Gly Gly Ile Ser Leu Leu Ser Glu
   1 5 10 15
<210> 86
   <211> 15
   <212> PRT
   <213> Künstliche Sequenz
<220>
   <223> Beschreibung der künstlichen Sequenz: HCMV IE-1 Fragment
<400> 86
   Thr Met Tyr Gly Gly Ile Ser Leu Leu Ser Glu Phe Cys Arg Val
   1 5 10 15
<210> 87
   <211> 15
   <212> PRT
   <213> Künstliche Sequenz
<220>
   <223> Beschreibung der künstlichen Sequenz: HCMV IE-1 Fragment
<400> 87
   Gly Ile Ser Leu Leu Ser Glu Phe Cys Arg Val Leu Cys Cys Tyr
   1 5 10 15
<210> 88
   <211> 15
   <212> PRT
   <213> Künstliche Sequenz
<220>
   <223> Beschreibung der künstlichen Sequenz: HCMV IE-1 Fragment
<400> 88
   Leu Ser Glu Phe Cys Arg Val Leu Cys Cys Tyr Val Leu Glu Glu
   1 5 10 15
<210> 89
   <211> 15
   <212> PRT
   <213> Künstliche Sequenz
<220>
   <223> Beschreibung der künstlichen Sequenz: HCMV IE-1 Fragment
<400> 89
   Cys Arg Val Leu Cys Cys Tyr Val Leu Glu Glu Thr Ser Val Met
   1 5 10 15
<210> 90
   <211> 15
   <212> PRT
   <213> Künstliche Sequenz
<220>
   <223> Beschreibung der künstlichen Sequenz: HCMV IE-1 Fragment
<400> 90
   Cys Cys Tyr Val Leu Glu Glu Thr Ser Val Met Leu Ala Lys Arg
   1 5 10 15
<210> 91
   <211> 15
   <212> PRT
   <213> Künstliche Sequenz
<220>
   <223> Beschreibung der künstlichen Sequenz: HCMV IE-1 Fragment
<400> 91
   Val Thr Lys Ala Thr Thr Phe Leu Gln Thr Met Leu Arg Lys Glu
   1 5 10 15
<210> 92
   <211> 15
   <212> PRT
   <213> Künstliche Sequenz
<220>
   <223> Beschreibung der künstlichen Sequenz: HCMV IE-1 Fragment
<400> 92
   Leu Glu Glu Thr Ser Val Met Leu Ala Lys Arg Pro Leu Ile Thr
   1 5 10 15
<210> 93
   <211> 15
   <212> PRT
   <213> Künstliche Sequenz
<220>
   <223> Beschreibung der künstlichen Sequenz: HCMV IE-1 Fragment
<400> 93
   Ser Val Met Leu Ala Lys Arg Pro Leu Ile Thr Lys Pro Glu Val
    1               5                  10                  15
<210> 94
   <211> 15
   <212> PRT
   <213> Künstliche Sequenz
<220>
   <223> Beschreibung der künstlichen Sequenz: HCMV IE-1 Fragment
<400> 94
   Ala Lys Arg Pro Leu Ile Thr Lys Pro Glu Val Ile Ser Val Met
    1               5                  10                  15
<210> 95
   <211> 15
   <212> PRT
   <213> Künstliche Sequenz
<220>
   <223> Beschreibung der künstlichen Sequenz: HCMV IE-1 Fragment
<400> 95
   Leu Ile Thr Lys Pro Glu Val Ile Ser Val Met Lys Arg Arg Ile
    1               5                  10                  15
<210> 96
   <211> 15
   <212> PRT
   <213> Künstliche Sequenz
<220>
   <223> Beschreibung der künstlichen Sequenz: HCMV IE-1 Fragment
<400> 96
   Pro Glu Val Ile Ser Val Met Lys Arg Arg Ile Glu Glu Ile Cys
    1               5                  10                  15
<210> 97
   <211> 15
   <212> PRT
   <213> Künstliche Sequenz
<220>
   <223> Beschreibung der künstlichen Sequenz: HCMV IE-1 Fragment
<400> 97
   Ser Val Met Lys Arg Arg Ile Glu Glu Ile Cys Met Lys Val Phe
   1               5                   10                  15
<210> 98
   <211> 15
   <212> PRT
   <213> Künstliche Sequenz
<220>
   <223> Beschreibung der künstlichen Sequenz: HCMV IE-1 Fragment
<400> 98
   Arg Arg Ile Glu Glu Ile Cys Met Lys Val Phe Ala Gln Tyr Ile
   1               5                   10                  15
<210> 99
   <211> 15
   <212> PRT
   <213> Künstliche Sequenz
<220>
   <223> Beschreibung der künstlichen Sequenz: HCMV IE-1 Fragment
<400> 99
   Glu Ile Cys Met Lys Val Phe Ala Gln Tyr Ile Leu Gly Ala Asp
   1               5                   10                  15
<210> 100
   <211> 15
   <212> PRT
   <213> Künstliche Sequenz
<220>
   <223> Beschreibung der künstlichen Sequenz: HCMV IE-1 Fragment
<400> 100
   Lys Val Phe Ala Gln Tyr Ile Leu Gly Ala Asp Pro Leu Arg Val
   1 5 10 15
<210> 101
   <211> 15
   <212> PRT
   <213> Künstliche Sequenz
<220>
   <223> Beschreibung der künstlichen Sequenz: HCMV IE-1 Fragment
<400> 101
   Gln Tyr Ile Leu Gly Ala Asp Pro Leu Arg Val Cys Ser Pro Ser
   1 5 10 15
<210> 102
   <211> 15
   <212> PRT
   <213> Künstliche Sequenz
<220>
   <223> Beschreibung der künstlichen Sequenz: HCMV IE-1 Fragment
<400> 102
   Thr Thr Phe Leu Gln Thr Met Leu Arg Lys Glu Val Asn Ser Gln
   1 5 10 15
<210> 103
   <211> 15
   <212> PRT
   <213> Künstliche Sequenz
<220>
   <223> Beschreibung der künstlichen Sequenz: HCMV IE-1 Fragment
<400> 103
   Gly Ala Asp Pro Leu Arg Val Cys Ser Pro Ser Val Asp Asp Leu
   1 5 10 15
<210> 104
   <211> 15
   <212> PRT
   <213> Künstliche Sequenz
<220>
   <223> Beschreibung der künstlichen Sequenz: HCMV IE-1 Fragment
<400> 104
   Leu Arg Val Cys Ser Pro Ser Val Asp Asp Leu Arg Ala Ile Ala
   1 5 10 15
<210> 105
   <211> 15
   <212> PRT
   <213> Künstliche Sequenz
<220>
   <223> Beschreibung der künstlichen Sequenz: HCMV IE-1 Fragment
<400> 105
   Ser Pro Ser Val Asp Asp Leu Arg Ala Ile Ala Glu Glu Ser Asp
   1 5 10 15
<210> 106
   <211> 15
   <212> PRT
   <213> Künstliche Sequenz
<220>
   <223> Beschreibung der künstlichen Sequenz: HCMV IE-1 Fragment
<400> 106
   Asp Asp Leu Arg Ala Ile Ala Glu Glu Ser Asp Glu Glu Glu Ala
   1 5 10 15
<210> 107
   <211> 15
   <212> PRT
   <213> Künstliche Sequenz
<220>
   <223> Beschreibung der künstlichen Sequenz: HCMV IE-1 Fragment
<400> 107
   Ala Ile Ala Glu Glu Ser Asp Glu Glu Glu Ala Ile Val Ala Tyr
   1 5 10 15
<210> 108
   <211> 15
   <212> PRT
   <213> Künstliche Sequenz
<220>
   <223> Beschreibung der künstlichen Sequenz: HCMV IE-1 Fragment
<400> 108
   Glu Ser Asp Glu Glu Glu Ala Ile Val Ala Tyr Thr Leu Ala Thr
   1 5 10 15
<210> 109
   <211> 15
   <212> PRT
   <213> Künstliche Sequenz
<220>
   <223> Beschreibung der künstlichen Sequenz: HCMV IE-1 Fragment
<400> 109
   Glu Glu Ala Ile Val Ala Tyr Thr Leu Ala Thr Ala Gly Val Ser
   1 5 10 15
<210> 110
   <211> 15
   <212> PRT
   <213> Künstliche Sequenz
<220>
   <223> Beschreibung der künstlichen Sequenz: HCMV IE-1 Fragment
<400> 110
   Val Ala Tyr Thr Leu Ala Thr Ala Gly Val Ser Ser Ser Asp Ser
   1 5 10 15
<210> 111
   <211> 15
   <212> PRT
   <213> Künstliche Sequenz
<220>
   <223> Beschreibung der künstlichen Sequenz: HCMV IE-1 Fragment
<400> 111
   Leu Ala Thr Ala Gly Val Ser Ser Ser Asp Ser Leu Val Ser Pro
   1 5 10 15
<210> 112
   <211> 15
   <212> PRT
   <213> Künstliche Sequenz
<220>
   <223> Beschreibung der künstlichen Sequenz: HCMV IE-1 Fragment
<400> 112
   Gly Val Ser Ser Ser Asp Ser Leu Val Ser Pro Pro Glu Ser Pro
   1 5 10 15
<210> 113
   <211> 15
   <212> PRT
   <213> Künstliche Sequenz
<220>
   <223> Beschreibung der künstlichen Sequenz: HCMV IE-1 Fragment
<400> 113
   Glu Glu Arg Glu Asp Thr Val Ser Val Lys Ser Glu Pro Val Ser
   1 5 10 15
<210> 114
   <211> 15
   <212> PRT
   <213> Künstliche Sequenz
<220>
   <223> Beschreibung der künstlichen Sequenz: HCMV IE-1 Fragment
<400> 114
   Asp Thr Val Ser Val Lys Ser Glu Pro Val Ser Glu Ile Glu Glu
   1 5 10 15
<210> 115
   <211> 15
   <212> PRT
   <213> Künstliche Sequenz
<220>
   <223> Beschreibung der künstlichen Sequenz: HCMV IE-1 Fragment
<400> 115
   Val Lys Ser Glu Pro Val Ser Glu Ile Glu Glu Val Ala Pro Glu
   1 5 10 15
<210> 116
   <211> 15
   <212> PRT
   <213> Künstliche Sequenz
<220>
   <223> Beschreibung der künstlichen Sequenz: HCMV IE-1 Fragment
<400> 116
   Pro Val Ser Glu Ile Glu Glu Val Ala Pro Glu Glu Glu Glu Asp
   1               5                   10                  15
<210> 117
   <211> 15
   <212> PRT
   <213> Künstliche Sequenz
<220>
   <223> Beschreibung der künstlichen Sequenz: HCMV IE-1 Fragment
<400> 117
   Ile Glu Glu Val Ala Pro Glu Glu Glu Glu Asp Gly Ala Glu Glu
   1               5                   10                  15
<210> 118
   <211> 15
   <212> PRT
   <213> Künstliche Sequenz
<220>
   <223> Beschreibung der künstlichen Sequenz: HCMV IE-1 Fragment
<400> 118
   Ala Pro Glu Glu Glu Glu Asp Gly Ala Glu Glu Pro Thr Ala Ser
   1               5                   10                  15
<210> 119
   <211> 15
   <212> PRT
   <213> Künstliche Sequenz
<220>
   <223> Beschreibung der künstlichen Sequenz: HCMV IE-1 Fragment
<400> 119
   Glu Glu Asp Gly Ala Glu Glu Pro Thr Ala Ser Gly Gly Lys Ser
   1               5                   10                  15
<210> 120
   <211> 15
   <212> PRT
   <213> Künstliche Sequenz
<220>
   <223> Beschreibung der künstlichen Sequenz: HCMV IE-1 Fragment
<400> 120
   Ala Glu Glu Pro Thr Ala Ser Gly Gly Lys Ser Thr His Pro Met
   1 5 10 15
<210> 121
   <211> 15
   <212> PRT
   <213> Künstliche Sequenz
<220>
   <223> Beschreibung der künstlichen Sequenz: HCMV IE-1 Fragment
<400> 121
   Thr Ala Ser Gly Gly Lys Ser Thr His Pro Met Val Thr Arg Ser
   1 5 10 15
<210> 122
   <211> 15
   <212> PRT
   <213> Künstliche Sequenz
<220>
   <223> Beschreibung der künstlichen Sequenz: HCMV IE-1 Fragment
<400> 122
   Gly Lys Ser Thr His Pro Met Val Thr Arg Ser Lys Ala Asp Gln
   1 5 10 15
<210> 123
   <211> 15
   <212> PRT
   <213> Künstliche Sequenz
<220>
   <223> Beschreibung der künstlichen Sequenz: HCMV pp65 Fragment
<400> 123
   Met Glu Ser Arg Gly Arg Arg Cys Pro Glu Met Ile Ser Val Leu
   1 5 10 15
<210> 124
   <211> 16
   <212> PRT
   <213> Künstliche Sequenz
<220>
   <223> Beschreibung der künstlichen Sequenz: HCMV pp65 Fragment
<400> 124
<210> 125
   <211> 17
   <212> PRT
   <213> Künstliche Sequenz
<220>
   <223> Beschreibung der künstlichen Sequenz: HCMV pp65 Fragment
<400> 125
<210> 126
   <211> 16
   <212> PRT
   <213> Künstliche Sequenz
<220>
   <223> Beschreibung der künstlichen Sequenz: HCMV pp65 Fragment
<400> 126
<210> 127
   <211> 16
   <212> PRT
   <213> Künstliche Sequenz
<220>
   <223> Beschreibung der künstlichen Sequenz: HCMV pp65 Fragment
<400> 127
<210> 128
   <211> 16
   <212> PRT
   <213> Künstliche Sequenz
<220>
   <223> Beschreibung der künstlichen Sequenz: HCMV pp65 Fragment
<400> 128
<210> 129
   <211> 16
   <212> PRT
   <213> Künstliche Sequenz
<220>
   <223> Beschreibung der künstlichen Sequenz: HCMV pp65 Fragment

<400> 129
<210> 130
   <211> 16
   <212> PRT
   <213> Künstliche Sequenz
<220>
   <223> Beschreibung der künstlichen Sequenz: HCMV pp65 Fragment
<400> 130
<210> 131
   <211> 16
   <212> PRT
   <213> Künstliche Sequenz
<220>
   <223> Beschreibung der künstlichen Sequenz: HCMV pp65 Fragment
<400> 131
<210> 132
   <211> 16
   <212> PRT
   <213> Künstliche Sequenz
<220>
   <223> Beschreibung der künstlichen Sequenz: HCMV pp65 Fragment
<400> 132
<210> 133
   <211> 16
   <212> PRT
   <213> Künstliche Sequenz
<220>
   <223> Beschreibung der künstlichen Sequenz: HCMV pp65 Fragment
<400> 133
<210> 134
   <211> 16
   <212> PRT
   <213> Künstliche Sequenz
<220>
   <223> Beschreibung der künstlichen Sequenz: HCMV pp65 Fragment
<400> 134
<210> 135
   <211> 15
   <212> PRT
   <213> Künstliche Sequenz
<220>
   <223> Beschreibung der künstlichen Sequenz: HCMV pp65 Fragment
<400> 135
   Leu Leu Gln Thr Gly Ile His Val Arg Val Ser Gln Pro Ser Leu
   1 5 10 15
<210> 136
   <211> 16
   <212> PRT
   <213> Künstliche Sequenz
<220>
   <223> Beschreibung der künstlichen Sequenz: HCMV pp65 Fragment
<400> 136
<210> 137
   <211> 15
   <212> PRT
   <213> Künstliche Sequenz
<220>
   <223> Beschreibung der künstlichen Sequenz: HCMV pp65 Fragment
<400> 137
   Ser Ser Ala Thr Ala Cys Thr Ser Gly Val Met Thr Arg Gly Arg
   1 5 10 15
<210> 138
   <211> 15
   <212> PRT
   <213> Künstliche Sequenz
<220>
   <223> Beschreibung der künstlichen Sequenz: HCMV pp65 Fragment
<400> 138
   Gly Ile His Val Arg Val Ser Gln Pro Ser Leu Ile Leu Val Ser
   1 5 10 15
<210> 139
   <211> 15
   <212> PRT
   <213> Künstliche Sequenz
<220>
   <223> Beschreibung der künstlichen Sequenz: HCMV pp65 Fragment
<400> 139
   Arg Val Ser Gln Pro Ser Leu Ile Leu Val Ser Gln Tyr Thr Pro
   1 5 10 15
<210> 140
   <211> 15
   <212> PRT
   <213> Künstliche Sequenz
<220>
   <223> Beschreibung der künstlichen Sequenz: HCMV pp65 Fragment
<400> 140
   Pro Ser Leu Ile Leu Val Ser Gln Tyr Thr Pro Asp Ser Thr Pro
   1 5 10 15
<210> 141
   <211> 15
   <212> PRT
   <213> Künstliche Sequenz
<220>
   <223> Beschreibung der künstlichen Sequenz: HCMV pp65 Fragment
<400> 141
   Leu Val Ser Gln Tyr Thr Pro Asp Ser Thr Pro Cys His Arg Gly
   1 5 10 15
<210> 142
   <211> 15
   <212> PRT
   <213> Künstliche Sequenz
<220>
   <223> Beschreibung der künstlichen Sequenz: HCMV pp65 Fragment
<400> 142
   Tyr Thr Pro Asp Ser Thr Pro Cys His Arg Gly Asp Asn Gln Leu
   1 5 10 15
<210> 143
   <211> 15
   <212> PRT
   <213> Künstliche Sequenz
<220>
   <223> Beschreibung der künstlichen Sequenz: HCMV pp65 Fragment
<400> 143
   Ser Thr Pro Cys His Arg Gly Asp Asn Gln Leu Gln Val Gln His
   1 5 10 15
<210> 144
   <211> 15
   <212> PRT
   <213> Künstliche Sequenz
<220>
   <223> Beschreibung der künstlichen Sequenz: HCMV pp65 Fragment
<400> 144
   His Arg Gly Asp Asn Gln Leu Gln Val Gln His Thr Tyr Phe Thr
   1 5 10 15
<210> 145
   <211> 15
   <212> PRT
   <213> Künstliche Sequenz
<220>
   <223> Beschreibung der künstlichen Sequenz: HCMV pp65 Fragment
<400> 145
   Asn Gln Leu Gln Val Gln His Thr Tyr Phe Thr Gly Ser Glu Val
   1 5 10 15
<210> 146
   <211> 15
   <212> PRT
   <213> Künstliche Sequenz
<220>
   <223> Beschreibung der künstlichen Sequenz: HCMV pp65 Fragment
<400> 146
   Gly Arg Arg Cys Pro Glu Met Ile Ser Val Leu Gly Pro Ile Ser
   1 5 10 15
<210> 147
   <211> 16
   <212> PRT
   <213> Künstliche Sequenz
<220>
   <223> Beschreibung der künstlichen Sequenz: HCMV pp65 Fragment
<400> 147
<210> 148
   <211> 15
   <212> PRT
   <213> Künstliche Sequenz
<220>
   <223> Beschreibung der künstlichen Sequenz: HCMV pp65 Fragment
<400> 148
   Tyr Phe Thr Gly Ser Glu Val Glu Asn Val Ser Val Asn Val His
   1               5                   10                  15
<210> 149
   <211> 15
   <212> PRT
   <213> Künstliche Sequenz
<220>
   <223> Beschreibung der künstlichen Sequenz: HCMV pp65 Fragment
<400> 149
   Ser Glu Val Glu Asn Val Ser Val Asn Val His Asn Pro Thr Gly
   1               5                   10                  15
<210> 150
   <211> 15
   <212> PRT
   <213> Künstliche Sequenz
<220>
   <223> Beschreibung der künstlichen Sequenz: HCMV pp65 Fragment
<400> 150
   Asn Val Ser Val Asn Val His Asn Pro Thr Gly Arg Ser Ile Cys
   1               5                   10                  15
<210> 151
   <211> 15
   <212> PRT
   <213> Künstliche Sequenz
<220>
   <223> Beschreibung der künstlichen Sequenz: HCMV pp65 Fragment
<400> 151
   Asn Val His Asn Pro Thr Gly Arg Ser Ile Cys Pro Ser Gln Glu
   1               5                   10                  15
<210> 152
   <211> 15
   <212> PRT
   <213> Künstliche Sequenz
<220>
   <223> Beschreibung der künstlichen Sequenz: HCMV pp65 Fragment
<400> 152
   Pro Thr Gly Arg Ser Ile Cys Pro Ser Gln Glu Pro Met Ser Ile
   1               5                   10                  15
<210> 153
   <211> 15
   <212> PRT
   <213> Künstliche Sequenz
<220>
   <223> Beschreibung der künstlichen Sequenz: HCMV pp65 Fragment
<400> 153
   Ser Ile Cys Pro Ser Gln Glu Pro Met Ser Ile Tyr Val Tyr Ala
   1               5                   10                  15
<210> 154
   <211> 15
   <212> PRT
   <213> Künstliche Sequenz
<220>
   <223> Beschreibung der künstlichen Sequenz: HCMV pp65 Fragment
<400> 154
   Ser Gln Glu Pro Met Ser Ile Tyr Val Tyr Ala Leu Pro Leu Lys
   1               5                   10                  15
<210> 155
   <211> 15
   <212> PRT
   <213> Künstliche Sequenz
<220>
   <223> Beschreibung der künstlichen Sequenz: HCMV pp65 Fragment
<400> 155
   Met Ser Ile Tyr Val Tyr Ala Leu Pro Leu Lys Met Leu Asn Ile
   1 5 10 15
<210> 156
   <211> 15
   <212> PRT
   <213> Künstliche Sequenz
<220>
   <223> Beschreibung der künstlichen Sequenz: HCMV pp65 Fragment
<400> 156
   Val Tyr Ala Leu Pro Leu Lys Met Leu Asn Ile Pro Ser Ile Asn
   1 5 10 15
<210> 157
   <211> 15
   <212> PRT
   <213> Künstliche Sequenz
<220>
   <223> Beschreibung der künstlichen Sequenz: HCMV pp65 Fragment
<400> 157
   Pro Glu Met Ile Ser Val Leu Gly Pro Ile Ser Gly His Val Leu
   1 5 10 15
<210> 158
   <211> 16
   <212> PRT
   <213> Künstliche Sequenz
<220>
   <223> Beschreibung der künstlichen Sequenz: HCMV pp65 Fragment
<400> 158
<210> 159
   <211> 15
   <212> PRT
   <213> Künstliche Sequenz
<220>
   <223> Beschreibung der künstlichen Sequenz: HCMV pp65 Fragment
<400> 159
   Leu Asn Ile Pro Ser Ile Asn Val His His Tyr Pro Ser Ala Ala
   1 5 10 15
<210> 160
   <211> 15
   <212> PRT
   <213> Künstliche Sequenz
<220>
   <223> Beschreibung der künstlichen Sequenz: HCMV pp65 Fragment
<400> 160
   Ser Ile Asn Val His His Tyr Pro Ser Ala Ala Glu Arg Lys His
   1 5 10 15
<210> 161
   <211> 15
   <212> PRT
   <213> Künstliche Sequenz
<220>
   <223> Beschreibung der künstlichen Sequenz: HCMV pp65 Fragment
<400> 161
   His His Tyr Pro Ser Ala Ala Glu Arg Lys His Arg His Leu Pro
   1 5 10 15
<210> 162
   <211> 15
   <212> PRT
   <213> Künstliche Sequenz
<220>
   <223> Beschreibung der künstlichen Sequenz: HCMV pp65 Fragment
<400> 162
   Ser Ala Ala Glu Arg Lys His Arg His Leu Pro Val Ala Asp Ala
   1 5 10 15
<210> 163
   <211> 15
   <212> PRT
   <213> Künstliche Sequenz
<220>
   <223> Beschreibung der künstlichen Sequenz: HCMV pp65 Fragment
<400> 163
   Arg Lys His Arg His Leu Pro Val Ala Asp Ala Val Ile His Ala
   1               5                   10                  15
<210> 164
   <211> 15
   <212> PRT
   <213> Künstliche Sequenz
<220>
   <223> Beschreibung der künstlichen Sequenz: HCMV pp65 Fragment
<400> 164
   His Leu Pro Val Ala Asp Ala Val Ile His Ala Ser Gly Lys Gln
   1               5                   10                  15
<210> 165
   <211> 15
   <212> PRT
   <213> Künstliche Sequenz
<220>
   <223> Beschreibung der künstlichen Sequenz: HCMV pp65 Fragment
<400> 165
   Ala Asp Ala Val Ile His Ala Ser Gly Lys Gln Met Trp Gln Ala
   1               5                   10                  15
<210> 166
   <211> 15
   <212> PRT
   <213> Künstliche Sequenz
<220>
   <223> Beschreibung der künstlichen Sequenz: HCMV pp65 Fragment
<400> 166
   Ile His Ala Ser Gly Lys Gln Met Trp Gln Ala Arg Leu Thr Val
   1               5                   10                  15
<210> 167
   <211> 15
   <212> PRT
   <213> Künstliche Sequenz
<220>
   <223> Beschreibung der künstlichen Sequenz: HCMV pp65 Fragment
<400> 167
   Gly Lys Gln Met Trp Gln Ala Arg Leu Thr Val Ser Gly Leu Ala
    1               5                  10                  15
<210> 168
   <211> 15
   <212> PRT
   <213> Künstliche Sequenz
<220>
   <223> Beschreibung der künstlichen Sequenz: HCMV pp65 Fragment
<400> 168
   Ser Val Leu Gly Pro Ile Ser Gly His Val Leu Lys Ala Val Phe
    1               5                  10                  15
<210> 169
   <211> 16
   <212> PRT
   <213> Künstliche Sequenz
<220>
   <223> Beschreibung der künstlichen Sequenz: HCMV pp65 Fragment
<400> 169
<210> 170
   <211> 15
   <212> PRT
   <213> Künstliche Sequenz
<220>
   <223> Beschreibung der künstlichen Sequenz: HCMV pp65 Fragment
<400> 170
   Leu Thr Val Ser Gly Leu Ala Trp Thr Arg Gln Gln Asn Gln Trp
    1               5                  10                  15
<210> 171
   <211> 15
   <212> PRT
   <213> Künstliche Sequenz
<220>
   <223> Beschreibung der künstlichen Sequenz: HCMV pp65 Fragment
<400> 171
   Gly Leu Ala Trp Thr Arg Gln Gln Asn Gln Trp Lys Glu Pro Asp
    1               5                  10                  15
<210> 172
   <211> 15
   <212> PRT
   <213> Künstliche Sequenz
<220>
   <223> Beschreibung der künstlichen Sequenz: HCMV pp65 Fragment
<400> 172
   Thr Arg Gln Gln Asn Gln Trp Lys Glu Pro Asp Val Tyr Tyr Thr
    1               5                  10                  15
<210> 173
   <211> 15
   <212> PRT
   <213> Künstliche Sequenz
<220>
   <223> Beschreibung der künstlichen Sequenz: HCMV pp65 Fragment
<400> 173
   Asn Gln Trp Lys Glu Pro Asp Val Tyr Tyr Thr Ser Ala Phe Val
    1               5                  10                  15
<210> 174
   <211> 15
   <212> PRT
   <213> Künstliche Sequenz
<220>
   <223> Beschreibung der künstlichen Sequenz: HCMV pp65 Fragment
<400> 174
   Glu Pro Asp Val Tyr Tyr Thr Ser Ala Phe Val Phe Pro Thr Lys
    1               5                  10                  15
<210> 175
   <211> 15
   <212> PRT
   <213> Künstliche Sequenz
<220>
   <223> Beschreibung der künstlichen Sequenz: HCMV pp65 Fragment
<400> 175
   Tyr Tyr Thr Ser Ala Phe Val Phe Pro Thr Lys Asp Val Ala Leu
   1 5 10 15
<210> 176
   <211> 15
   <212> PRT
   <213> Künstliche Sequenz
<220>
   <223> Beschreibung der künstlichen Sequenz: HCMV pp65 Fragment
<400> 176
   Ala Phe Val Phe Pro Thr Lys Asp Val Ala Leu Arg His Val Val
   1 5 10 15
<210> 177
   <211> 15
   <212> PRT
   <213> Künstliche Sequenz
<220>
   <223> Beschreibung der künstlichen Sequenz: HCMV pp65 Fragment
<400> 177
   Pro Thr Lys Asp Val Ala Leu Arg His Val Val Cys Ala His Glu
   1 5 10 15
<210> 178
   <211> 15
   <212> PRT
   <213> Künstliche Sequenz
<220>
   <223> Beschreibung der künstlichen Sequenz: HCMV pp65 Fragment
<400> 178
   Val Ala Leu Arg His Val Val Cys Ala His Glu Leu Val Cys Ser
   1 5 10 15
<210> 179
   <211> 15
   <212> PRT
   <213> Künstliche Sequenz
<220>
   <223> Beschreibung der künstlichen Sequenz: HCMV pp65 Fragment
<400> 179
   Pro Ile Ser Gly His Val Leu Lys Ala Val Phe Ser Arg Gly Asp
   1               5                   10                  15
<210> 180
   <211> 16
   <212> PRT
   <213> Künstliche Sequenz
<220>
   <223> Beschreibung der künstlichen Sequenz : HCMV pp65 Fragment
<400> 180
<210> 181
   <211> 15
   <212> PRT
   <213> Künstliche Sequenz
<220>
   <223> Beschreibung der künstlichen Sequenz: HCMV pp65 Fragment
<400> 181
   Ala His Glu Leu Val Cys Ser Met Glu Asn Thr Arg Ala Thr Lys
   1               5                   10                  15
<210> 182
   <211> 15
   <212> PRT
   <213> Künstliche Sequenz
<220>
   <223> Beschreibung der künstlichen Sequenz: HCMV pp65 Fragment
<400> 182
   Val Cys Ser Met Glu Asn Thr Arg Ala Thr Lys Met Gln Val Ile
   1               5                   10                  15
<210> 183
   <211> 15
   <212> PRT
   <213> Künstliche Sequenz
<220>
   <223> Beschreibung der künstlichen Sequenz: HCMV pp65 Fragment
<400> 183
   Glu Asn Thr Arg Ala Thr Lys Met Gln Val Ile Gly Asp Gln Tyr
   1 5 10 15
<210> 184
   <211> 15
   <212> PRT
   <213> Künstliche Sequenz
<220>
   <223> Beschreibung der künstlichen Sequenz : HCMV pp65 Fragment
<400> 184
   Ala Thr Lys Met Gln Val Ile Gly Asp Gln Tyr Val Lys Val Tyr
   1 5 10 15
<210> 185
   <211> 15
   <212> PRT
   <213> Künstliche Sequenz
<220>
   <223> Beschreibung der künstlichen Sequenz: HCMV pp65 Fragment
<400> 185
   Gln Val Ile Gly Asp Gln Tyr Val Lys Val Tyr Leu Glu Ser Phe
   1 5 10 15
<210> 186
   <211> 15
   <212> PRT
   <213> Künstliche Sequenz
<220>
   <223> Beschreibung der künstlichen Sequenz: HCMV pp65 Fragment
<400> 186
   Asp Gln Tyr Val Lys Val Tyr Leu Glu Ser Phe Cys Glu Asp Val
   1 5 10 15
<210> 187
   <211> 15
   <212> PRT
   <213> Künstliche Sequenz
<220>
   <223> Beschreibung der künstlichen Sequenz: HCMV pp65 Fragment
<400> 187
   Lys Val Tyr Leu Glu Ser Phe Cys Glu Asp Val Pro Ser Gly Lys
   1 5 10 15
<210> 188
   <211> 15
   <212> PRT
   <213> Künstliche Sequenz
<220>
   <223> Beschreibung der künstlichen Sequenz: HCMV pp65 Fragment
<400> 188
   Glu Ser Phe Cys Glu Asp Val Pro Ser Gly Lys Leu Phe Met His
   1 5 10 15
<210> 189
   <211> 15
   <212> PRT
   <213> Künstliche Sequenz
<220>
   <223> Beschreibung der künstlichen Sequenz: HCMV pp65 Fragment
<400> 189
   Glu Asp Val Pro Ser Gly Lys Leu Phe Met His Val Thr Leu Gly
   1 5 10 15
<210> 190
   <211> 15
   <212> PRT
   <213> Künstliche Sequenz
<220>
   <223> Beschreibung der künstlichen Sequenz: HCMV pp65 Fragment
<400> 190
   His Val Leu Lys Ala Val Phe Ser Arg Gly Asp Thr Pro Val Leu
   1 5 10 15
<210> 191
   <211> 16
   <212> PRT
   <213> Künstliche Sequenz
<220>
   <223> Beschreibung der künstlichen Sequenz: HCMV pp65 Fragment
<400> 191
<210> 192
   <211> 15
   <212> PRT
   <213> Künstliche Sequenz
<220>
   <223> Beschreibung der künstlichen Sequenz: HCMV pp65 Fragment
<400> 192
   Phe Met His Val Thr Leu Gly Ser Asp Val Glu Glu Asp Leu Thr
   1 5 10 15
<210> 193
   <211> 15
   <212> PRT
   <213> Künstliche Sequenz
<220>
   <223> Beschreibung der künstlichen Sequenz: HCMV pp65 Fragment
<400> 193
   Thr Leu Gly Ser Asp Val Glu Glu Asp Leu Thr Met Thr Arg Asn
   1 5 10 15
<210> 194
   <211> 15
   <212> PRT
   <213> Künstliche Sequenz
<220>
   <223> Beschreibung der künstlichen Sequenz: HCMV pp65 Fragment
<400> 194
   Asp Val Glu Glu Asp Leu Thr Met Thr Arg Asn Pro Gln Pro Phe
   1               5                   10                  15
<210> 195
   <211> 15
   <212> PRT
   <213> Künstliche Sequenz
<220>
   <223> Beschreibung der künstlichen Sequenz: HCMV pp65 Fragment
<400> 195
   Asp Leu Thr Met Thr Arg Asn Pro Gln Pro Phe Met Arg Pro His
   1               5                   10                  15
<210> 196
   <211> 15
   <212> PRT
   <213> Künstliche Sequenz
<220>
   <223> Beschreibung der künstlichen Sequenz: HCMV pp65 Fragment
<400> 196
   Thr Arg Asn Pro Gln Pro Phe Met Arg Pro His Glu Arg Asn Gly
   1               5                   10                  15
<210> 197
   <211> 15
   <212> PRT
   <213> Künstliche Sequenz
<220>
   <223> Beschreibung der künstlichen Sequenz: HCMV pp65 Fragment
<400> 197
   Gln Pro Phe Met Arg Pro His Glu Arg Asn Gly Phe Thr Val Leu
   1               5                   10                  15
<210> 198
   <211> 15
   <212> PRT
   <213> Künstliche Sequenz
<220>
   <223> Beschreibung der künstlichen Sequenz: HCMV pp65 Fragment
<400> 198
   Arg Pro His Glu Arg Asn Gly Phe Thr Val Leu Cys Pro Lys Asn
   1 5 10 15
<210> 199
   <211> 15
   <212> PRT
   <213> Künstliche Sequenz
<220>
   <223> Beschreibung der künstlichen Sequenz: HCMV pp65 Fragment
<400> 199
   Arg Asn Gly Phe Thr Val Leu Cys Pro Lys Asn Met Ile Ile Lys
   1 5 10 15
<210> 200
   <211> 15
   <212> PRT
   <213> Künstliche Sequenz
<220>
   <223> Beschreibung der künstlichen Sequenz: HCMV pp65 Fragment
<400> 200
   Thr Val Leu Cys Pro Lys Asn Met Ile Ile Lys Pro Gly Lys Ile
   1 5 10 15
<210> 201
   <211> 15
   <212> PRT
   <213> Künstliche Sequenz
<220>
   <223> Beschreibung der künstlichen Sequenz: HCMV pp65 Fragment
<400> 201
   Ala Val Phe Ser Arg Gly Asp Thr Pro Val Leu Pro His Glu Thr
   1 5 10 15
<210> 202
   <211> 16
   <212> PRT
   <213> Künstliche Sequenz
<220>
   <223> Beschreibung der künstlichen Sequenz: HCMV pp65 Fragment
<400> 202
<210> 203
   <211> 15
   <212> PRT
   <213> Künstliche Sequenz
<220>
   <223> Beschreibung der künstlichen Sequenz: HCMV pp65 Fragment
<400> 203
   Ile Ile Lys Pro Gly Lys Ile Ser His Ile Met Leu Asp Val Ala
   1               5                   10                  15
<210> 204
   <211> 15
   <212> PRT
   <213> Künstliche Sequenz
<220>
   <223> Beschreibung der künstlichen Sequenz: HCMV pp65 Fragment
<400> 204
   Gly Lys Ile Ser His Ile Met Leu Asp Val Ala Phe Thr Ser His
   1               5                   10                  15
<210> 205
   <211> 15
   <212> PRT
   <213> Künstliche Sequenz
<220>
   <223> Beschreibung der künstlichen Sequenz: HCMV pp65 Fragment
<400> 205
   His Ile Met Leu Asp Val Ala Phe Thr Ser His Glu His Phe Gly
   1               5                   10                  15
<210> 206
   <211> 15
   <212> PRT
   <213> Künstliche Sequenz
<220>
   <223> Beschreibung der künstlichen Sequenz: HCMV pp65 Fragment
<400> 206
   Asp Val Ala Phe Thr Ser His Glu His Phe Gly Leu Leu Cys Pro
   1 5 10 15
<210> 207
   <211> 15
   <212> PRT
   <213> Künstliche Sequenz
<220>
   <223> Beschreibung der künstlichen Sequenz: HCMV pp65 Fragment
<400> 207
   Thr Ser His Glu His Phe Gly Leu Leu Cys Pro Lys Ser Ile Pro
   1 5 10 15
<210> 208
   <211> 15
   <212> PRT
   <213> Künstliche Sequenz
<220>
   <223> Beschreibung der künstlichen Sequenz: HCMV pp65 Fragment
<400> 208
   His Phe Gly Leu Leu Cys Pro Lys Ser Ile Pro Gly Leu Ser Ile
   1 5 10 15
<210> 209
   <211> 15
   <212> PRT
   <213> Künstliche Sequenz
<220>
   <223> Beschreibung der künstlichen Sequenz: HCMV pp65 Fragment
<400> 209
   Leu Cys Pro Lys Ser Ile Pro Gly Leu Ser Ile Ser Gly Asn Leu
   1 5 10 15
<210> 210
   <211> 15
   <212> PRT
   <213> Künstliche Sequenz
<220>
   <223> Beschreibung der künstlichen Sequenz: HCMV pp65 Fragment
<400> 210
   Ser Ile Pro Gly Leu Ser Ile Ser Gly Asn Leu Leu Met Asn Gly
   1               5                   10                  15
<210> 211
   <211> 15
   <212> PRT
   <213> Künstliche Sequenz
<220>
   <223> Beschreibung der künstlichen Sequenz: HCMV pp65 Fragment
<400> 211
   Leu Ser Ile Ser Gly Asn Leu Leu Met Asn Gly Gln Gln Ile Phe
   1               5                   10                  15
<210> 212
   <211> 15
   <212> PRT
   <213> Künstliche Sequenz
<220>
   <223> Beschreibung der künstlichen Sequenz: HCMV pp65 Fragment
<400> 212
   Arg Gly Asp Thr Pro Val Leu Pro His Glu Thr Arg Leu Leu Gln
   1               5                   10                  15
<210> 213
   <211> 16
   <212> PRT
   <213> Künstliche Sequenz
<220>
   <223> Beschreibung der künstlichen Sequenz: HCMV pp65 Fragment
<400> 213
<210> 214
   <211> 15
   <212> PRT
   <213> Künstliche Sequenz
<220>
   <223> Beschreibung der künstlichen Sequenz: HCMV pp65 Fragment
<400> 214
   Met Asn Gly Gln Gln Ile Phe Leu Glu Val Gln Ala Ile Arg Glu
   1               5                   10                  15
<210> 215
   <211> 15
   <212> PRT
   <213> Künstliche Sequenz
<220>
   <223> Beschreibung der künstlichen Sequenz: HCMV pp65 Fragment
<400> 215
   Gln Ile Phe Leu Glu Val Gln Ala Ile Arg Glu Thr Val Glu Leu
   1               5                   10                  15
<210> 216
   <211> 15
   <212> PRT
   <213> Künstliche Sequenz
<220>
   <223> Beschreibung der künstlichen Sequenz: HCMV pp65 Fragment
<400> 216
   Glu Val Gln Ala Ile Arg Glu Thr Val Glu Leu Arg Gln Tyr Asp
   1               5                   10                  15
<210> 217
   <211> 15
   <212> PRT
   <213> Künstliche Sequenz
<220>
   <223> Beschreibung der künstlichen Sequenz: HCMV pp65 Fragment
<400> 217
   Ile Arg Glu Thr Val Glu Leu Arg Gln Tyr Asp Pro Val Ala Ala
   1 5 10 15
<210> 218
   <211> 15
   <212> PRT
   <213> Künstliche Sequenz
<220>
   <223> Beschreibung der künstlichen Sequenz: HCMV pp65 Fragment
<400> 218
   Val Glu Leu Arg Gln Tyr Asp Pro Val Ala Ala Leu Phe Phe Phe
   1 5 10 15
<210> 219
   <211> 15
   <212> PRT
   <213> Künstliche Sequenz
<220>
   <223> Beschreibung der künstlichen Sequenz: HCMV pp65 Fragment
<400> 219
   Gln Tyr Asp Pro Val Ala Ala Leu Phe Phe Phe Asp Ile Asp Leu
   1 5 10 15
<210> 220
   <211> 15
   <212> PRT
   <213> Künstliche Sequenz
<220>
   <223> Beschreibung der künstlichen Sequenz: HCMV pp65 Fragment
<400> 220
   Val Ala Ala Leu Phe Phe Phe Asp Ile Asp Leu Leu Leu Gln Arg
   1 5 10 15
<210> 221
   <211> 15
   <212> PRT
   <213> Künstliche Sequenz
<220>
   <223> Beschreibung der künstlichen Sequenz: HCMV pp65 Fragment
<400> 221
   Phe Phe Phe Asp Ile Asp Leu Leu Leu Gln Arg Gly Pro Gln Tyr
   1 5 10 15
<210> 222
   <211> 15
   <212> PRT
   <213> Künstliche Sequenz
<220>
   <223> Beschreibung der künstlichen Sequenz: HCMV pp65 Fragment
<400> 222
   Ile Asp Leu Leu Leu Gln Arg Gly Pro Gln Tyr Ser Glu His Pro
   1 5 10 15
<210> 223
   <211> 15
   <212> PRT
   <213> Künstliche Sequenz
<220>
   <223> Beschreibung der künstlichen Sequenz: HCMV pp65 Fragment
<400> 223
   Pro Val Leu Pro His Glu Thr Arg Leu Leu Gln Thr Gly Ile His
   1 5 10 15
<210> 224
   <211> 16
   <212> PRT
   <213> Künstliche Sequenz
<220>
   <223> Beschreibung der künstlichen Sequenz: HCMV pp65 Fragment
<400> 224
<210> 225
   <211> 15
   <212> PRT
   <213> Künstliche Sequenz
<220>
   <223> Beschreibung der künstlichen Sequenz: HCMV pp65 Fragment
<400> 225
   Pro Gln Tyr Ser Glu His Pro Thr Phe Thr Ser Gln Tyr Arg Ile
   1 5 10 15
<210> 226
   <211> 15
   <212> PRT
   <213> Künstliche Sequenz
<220>
   <223> Beschreibung der künstlichen Sequenz: HCMV pp65 Fragment
<400> 226
   Glu His Pro Thr Phe Thr Ser Gln Tyr Arg Ile Gln Gly Lys Leu
   1 5 10 15
<210> 227
   <211> 15
   <212> PRT
   <213> Künstliche Sequenz
<220>
   <223> Beschreibung der künstlichen Sequenz: HCMV pp65 Fragment
<400> 227
   Phe Thr Ser Gln Tyr Arg Ile Gln Gly Lys Leu Glu Tyr Arg His
   1 5 10 15
<210> 228
   <211> 15
   <212> PRT
   <213> Künstliche Sequenz
<220>
   <223> Beschreibung der künstlichen Sequenz: HCMV pp65 Fragment
<400> 228
   Tyr Arg Ile Gln Gly Lys Leu Glu Tyr Arg His Thr Trp Asp Arg
   1 5 10 15
<210> 229
   <211> 15
   <212> PRT
   <213> Künstliche Sequenz
<220>
   <223> Beschreibung der künstlichen Sequenz: HCMV pp65 Fragment
<400> 229
   Gly Lys Leu Glu Tyr Arg His Thr Trp Asp Arg His Asp Glu Gly
   1               5                   10                  15
<210> 230
   <211> 15
   <212> PRT
   <213> Künstliche Sequenz
<220>
   <223> Beschreibung der künstlichen Sequenz: HCMV pp65 Fragment
<400> 230
   Tyr Arg His Thr Trp Asp Arg His Asp Glu Gly Ala Ala Gln Gly
   1               5                   10                  15
<210> 231
   <211> 15
   <212> PRT
   <213> Künstliche Sequenz
<220>
   <223> Beschreibung der künstlichen Sequenz: HCMV pp65 Fragment
<400> 231
   Trp Asp Arg His Asp Glu Gly Ala Ala Gln Gly Asp Asp Asp Val
   1               5                   10                  15
<210> 232
   <211> 15
   <212> PRT
   <213> Künstliche Sequenz
<220>
   <223> Beschreibung der künstlichen Sequenz: HCMV pp65 Fragment
<400> 232
   Asp Glu Gly Ala Ala Gln Gly Asp Asp Asp Val Trp Thr Ser Gly
   1               5                   10                  15
<210> 233
   <211> 15
   <212> PRT
   <213> Künstliche Sequenz
<220>
   <223> Beschreibung der künstlichen Sequenz: HCMV pp65 Fragment
<400> 233
   Ala Gln Gly Asp Asp Asp Val Trp Thr Ser Gly Ser Asp Ser Asp
   1 5 10 15
<210> 234
   <211> 15
   <212> PRT
   <213> Künstliche Sequenz
<220>
   <223> Beschreibung der künstlichen Sequenz: HCMV pp65 Fragment
<400> 234
   Ala Cys Thr Ser Gly Val Met Thr Arg Gly Arg Leu Lys Ala Glu
   1 5 10 15
<210> 235
   <211> 15
   <212> PRT
   <213> Künstliche Sequenz
<220>
   <223> Beschreibung der künstlichen Sequenz: HCMV pp65 Fragment
<400> 235
   Gly Val Met Thr Arg Gly Arg Leu Lys Ala Glu Ser Thr Val Ala
   1 5 10 15
<210> 236
   <211> 15
   <212> PRT
   <213> Künstliche Sequenz
<220>
   <223> Beschreibung der künstlichen Sequenz: HCMV pp65 Fragment
<400> 236
   Arg Gly Arg Leu Lys Ala Glu Ser Thr Val Ala Pro Glu Glu Asp
   1 5 10 15
<210> 237
   <211> 15
   <212> PRT
   <213> Künstliche Sequenz
<220>
   <223> Beschreibung der künstlichen Sequenz: HCMV pp65 Fragment
<400> 237
   Lys Ala Glu Ser Thr Val Ala Pro Glu Glu Asp Thr Asp Glu Asp
   1               5                   10                  15
<210> 238
   <211> 15
   <212> PRT
   <213> Künstliche Sequenz
<220>
   <223> Beschreibung der künstlichen Sequenz: HCMV pp65 Fragment
<400> 238
   Thr Val Ala Pro Glu Glu Asp Thr Asp Glu Asp Ser Asp Asn Glu
   1               5                   10                  15
<210> 239
   <211> 15
   <212> PRT
   <213> Künstliche Sequenz
<220>
   <223> Beschreibung der künstlichen Sequenz: HCMV pp65 Fragment
<400> 239
   Glu Glu Asp Thr Asp Glu Asp Ser Asp Asn Glu Ile His Asn Pro
   1               5                   10                  15
<210> 240
   <211> 15
   <212> PRT
   <213> Künstliche Sequenz
<220>
   <223> Beschreibung der künstlichen Sequenz: HCMV pp65 Fragment
<400> 240
   Asp Glu Asp Ser Asp Asn Glu Ile His Asn Pro Ala Val Phe Thr
   1               5                   10                  15
<210> 241
   <211> 15
   <212> PRT
   <213> Künstliche Sequenz
<220>
   <223> Beschreibung der künstlichen Sequenz: HCMV pp65 Fragment
<400> 241
   Asp Asn Glu Ile His Asn Pro Ala Val Phe Thr Trp Pro Pro Trp
   1 5 10 15
<210> 242
   <211> 16
   <212> PRT
   <213> Künstliche Sequenz
<220>
   <223> Beschreibung der künstlichen Sequenz: HCMV pp65 Fragment
<400> 242
<210> 243
   <211> 15
   <212> PRT
   <213> Künstliche Sequenz
<220>
   <223> Beschreibung der künstlichen Sequenz: HCMV pp65 Fragment
<400> 243
   Val Phe Thr Trp Pro Pro Trp Gln Ala Gly Ile Leu Ala Arg Asn
   1 5 10 15
<210> 244
   <211> 15
   <212> PRT
   <213> Künstliche Sequenz
<220>
   <223> Beschreibung der künstlichen Sequenz: HCMV pp65 Fragment
<400> 244
   Pro Pro Trp Gln Ala Gly Ile Leu Ala Arg Asn Leu Val Pro Met
   1 5 10 15
<210> 245
   <211> 15
   <212> PRT
   <213> Künstliche Sequenz
<220>
   <223> Beschreibung der künstlichen Sequenz: HCMV pp65 Fragment
<400> 245
   Ala Gly Ile Leu Ala Arg Asn Leu Val Pro Met Val Ala Thr Val
   1 5 10 15
<210> 246
   <211> 15
   <212> PRT
   <213> Künstliche Sequenz
<220>
   <223> Beschreibung der künstlichen Sequenz: HCMV pp65 Fragment
<400> 246
   Ala Arg Asn Leu Val Pro Met Val Ala Thr Val Gln Gly Gln Asn
   1 5 10 15
<210> 247
   <211> 15
   <212> PRT
   <213> Künstliche Sequenz
<220>
   <223> Beschreibung der künstlichen Sequenz: HCMV pp65 Fragment
<400> 247
   Val Pro Met Val Ala Thr Val Gln Gly Gln Asn Leu Lys Tyr Gln
   1 5 10 15
<210> 248
   <211> 15
   <212> PRT
   <213> Künstliche Sequenz
<220>
   <223> Beschreibung der künstlichen Sequenz: HCMV pp65 Fragment
<400> 248
   Ala Thr Val Gln Gly Gln Asn Leu Lys Tyr Gln Glu Phe Phe Trp
   1 5 10 15
<210> 249
   <211> 15
   <212> PRT
   <213> Künstliche Sequenz
<220>
   <223> Beschreibung der künstlichen Sequenz: HCMV pp65 Fragment
<400> 249
   Gly Gln Asn Leu Lys Tyr Gln Glu Phe Phe Trp Asp Ala Asn Asp
   1 5 10 15
<210> 250
   <211> 15
   <212> PRT
   <213> Künstliche Sequenz
<220>
   <223> Beschreibung der künstlichen Sequenz: HCMV pp65 Fragment
<400> 250
   Lys Tyr Gln Glu Phe Phe Trp Asp Ala Asn Asp Ile Tyr Arg Ile
   1 5 10 15
<210> 251
   <211> 15
   <212> PRT
   <213> Künstliche Sequenz
<220>
   <223> Beschreibung der künstlichen Sequenz: HCMV pp65 Fragment
<400> 251
   Phe Phe Trp Asp Ala Asn Asp Ile Tyr Arg Ile Phe Ala Glu Leu
   1 5 10 15
<210> 252
   <211> 16
   <212> PRT
   <213> Künstliche Sequenz
<220>
   <223> Beschreibung der künstlichen Sequenz: HCMV pp65 Fragment
<400> 252
<210> 253
   <211> 15
   <212> PRT
   <213> Künstliche Sequenz
<220>
   <223> Beschreibung der künstlichen Sequenz: HCMV pp65 Fragment
<400> 253
   Tyr Arg Ile Phe Ala Glu Leu Glu Gly Val Trp Gln Pro Ala Ala
   1 5 10 15
<210> 254
   <211> 15
   <212> PRT
   <213> Künstliche Sequenz
<220>
   <223> Beschreibung der künstlichen Sequenz: HCMV pp65 Fragment
<400> 254
   Ala Glu Leu Glu Gly Val Trp Gln Pro Ala Ala Gln Pro Lys Arg
   1 5 10 15
<210> 255
   <211> 15
   <212> PRT
   <213> Künstliche Sequenz
<220>
   <223> Beschreibung der künstlichen Sequenz: HCMV pp65 Fragment
<400> 255
   Gly Val Trp Gln Pro Ala Ala Gln Pro Lys Arg Arg Arg His Arg
   1 5 10 15
<210> 256
   <211> 15
   <212> PRT
   <213> Künstliche Sequenz
<220>
   <223> Beschreibung der künstlichen Sequenz: HCMV pp65 Fragment
<400> 256
   Pro Ala Ala Gln Pro Lys Arg Arg Arg His Arg Gln Asp Ala Leu
   1 5 10 15
<210> 257
   <211> 15
   <212> PRT
   <213> Künstliche Sequenz
<220>
   <223> Beschreibung der künstlichen Sequenz: HCMV pp65 Fragment
<400> 257
   Pro Lys Arg Arg Arg His Arg Gln Asp Ala Leu Pro Gly Pro Cys
   1               5                   10                  15
<210> 258
   <211> 15
   <212> PRT
   <213> Künstliche Sequenz
<220>
   <223> Beschreibung der künstlichen Sequenz: HCMV pp65 Fragment
<400> 258
   Arg His Arg Gln Asp Ala Leu Pro Gly Pro Cys Ile Ala Ser Thr
   1               5                   10                  15
<210> 259
   <211> 15
   <212> PRT
   <213> Künstliche Sequenz
<220>
   <223> Beschreibung der künstlichen Sequenz: HCMV pp65 Fragment
<400> 259
   Asp Ala Leu Pro Gly Pro Cys Ile Ala Ser Thr Pro Lys Lys His
   1               5                   10                  15
<210> 260
   <211> 15
   <212> PRT
   <213> Künstliche Sequenz
<220>
   <223> Beschreibung der künstlichen Sequenz: HCMV pp65 Fragment
<400> 260
   Leu Pro Gly Pro Cys Ile Ala Ser Thr Pro Lys Lys His Arg Gly
    1               5                  10                  15

## Beschreibung

Die Erfindung betrifft ein Verfahren zur antigen-spezifischen Stimulation von CD8- und/oder CD4-T-Lymphozyten mit synthetischen Peptidbibliotheken, umfassend die folgenden Schritte:
(a) Unterteilen der Gesamt-Aminosäure-Sequenz des gesamten Proteins in Proteinfragmente mit Teil-Aminosäuresequenzen,
(b) Synthetisieren einer Peptidbibliothek, enthaltend diese Proteinfragmente;
(c) Inkubieren einer Suspension mit den zu stimulierenden CD8-und/oder CD4-T-Lymphozyten mit allen Proteinfragmenten der Peptidbibliothek in einem einzigen Kulturansatz.

Das Verfahren kann sowohl zur Immunstimulation von T-Lymphozyten von Säugern, insbesondere von Menschen, als auch zum Nachweis einer T-Zell-Immunantwort um festzustellen, ob ein Säuger, insbesondere ein Mensch, zuvor gegen wenigstens ein Proteinfragment des gesamten Proteins von Schritt (a) mit seinem Immunsystem geantwortet hat und wie stark diese Antwort ist, eingesetzt werden.

### Hintergrund der Erfindung

Die Immunantwort von CD8-T-Lymphozyten gegen Proteinantigene lässt sich mit bekannten Methoden nur mit großem Aufwand erfassen. Sie ist abhängig von der Präsentation der von diesen Antigenen abgeleiteten Epitope auf zellständigen MHC-Klasse-I-Molekülen und kann über die Messung einer durch Exposition induzierten zytotoxischen Reaktion gemessen werden. Diese Versuchsanordnung ist üblich und nimmt eine bis mehrere Wochen in Anspruch, wobei die CD8-T-Lymphozyten in einer geeigneten Zellkultur mit dem Antigen stimuliert werden müssen und dann in einem Zytotoxizitätstest mit geeigneten Zielzellen (Target-Cells) die mit Peptiden von diesem Antigen beladen wurden oder mit diesem Antigen oder Teilen davon transfiziert wurden, inkubiert werden. Die Induktion einer Antwort der CD8 T-Lymphozyten wird am Ausmaß der Zerstörung der Zielzellen gemessen, was geeignete Kontrollen erfordert und einen großen experimentellen und zeitlichen Aufwand beinhaltet.

Die Erfassung der Immunantwort von CD4-T-Lymphozyten gegen Proteinantigene ist etwas weniger kompliziert. Die Antwort von CD4 T-Lymphozyten gegen Proteinantigene ist abhängig von der Präsentation der von diesen Antigenen abgeleiteten Epitope auf zellständigen MHC-Klasse-II-Molekülen und kann über die Proliferation dieser Zellen in Gegenwart des Antigens bzw. nach Exposition mit diesem Antigen z.B. über den Einbau von tritiiertem Thymidin gemessen werden. Diese Versuchsanordnung ist üblich und nimmt mehrere Tage bis zu einer Woche oder länger in Anspruch. Das Vorhandensein einer CD4-T-Lymphozyten-Antwort gegen Proteinantigene kann weiterhin in einem bekannten Verfahren gemessen werden, indem eine CD4-T-Lymphozyten enthaltende Suspension mit dem entsprechenden Protein inkubiert wird und anschließend die CD4-T-Lymphozyteninduktion über das Vorhandensein intrazellulärer Zytokine durchflusszytometrisch erfasst wird.

Das Vorhandensein einer CD8-T-Lymphozyten oder CD4-Lymphozyten-Antwort gegen einzelne Epitope kann weiterhin in einem bekannten Verfahren gemessen werden, indem eine CD8- und/oder CD4- T-Lymphozyten enthaltende Suspension mit Peptiden aus diesem Protein inkubiert wird und anschließend die CD8- oder CD4-T-Lymphozyteninduktion über das Vorhandensein intrazellulärer Zytokine durchflusszytometrisch erfasst wird. Dabei macht man sich zunutze, dass Peptide unter Umgehung der intrazellulären Prozessierung direkt von außen auf die zellständigen MHC-Klasse-I oder MHC-Klasse-II-Moleküle aufgeladen werden können. Durch eine geeignete Anordnung von Peptiden in Gruppen kann bei diesem Verfahren erreicht werden dass stimulierende Peptide identifiziert werden können und somit Epitope bestimmt werden können. Die in dieser Art verwendete Anordnung verteilt alle in Frage kommenden Epitope auf mehrere, meistens zahlreiche Ansätze, so dass sich ermitteln lässt, ob einzelne Peptide aus diesem Protein eine T-Lymphozyten-Antwort induzieren können und sich ermitteln lässt, welche der in den einzelnen Gruppen vorkommenden Peptide zu dieser Stimulation geführt haben (dies wird beschrieben bei F. Kern et al., Journal of Virology, Oktober 1999, p. 8179-8184 und in WO 99/36568).

Diese Anordnung gestattet aber weder, systematisch *in einer einzigen Messung* mit entsprechendem Kontrollansatz zu bestimmen, ob überhaupt eine T-Lymphozyten-Antwort gegen das Protein vorhanden ist, noch, auszusagen, wie groß die Antwort (der Anteil der reaktiven in % der gesamten CD8- oder CD4-T-Lymphozyten) gegen dieses Protein insgesamt ist. Dazu wären bei der gebräuchlichen Anordnung bei diesem Verfahren zur Identifikation von Epitopen mehrere Stimulations- und Messansätze notwendig, abhängig davon, wie viele Peptide verwendet werden. Die in der Literatur beschriebene Anwendung, hat zum Ziel die präzise Identifikation von Epitopen und verwendet deshalb Gruppen von Peptiden, deren Größe so gewählt wird, dass bei Feststellung einer stimulierenden Wirkung einer Peptidgruppe möglichst wenige einzelne Peptide getestet werden müssen. Je kleiner man aber die Gruppengröße wählt, desto mehr Gruppen muss man testen. Als günstigste Variante wählt man deshalb bei diesem Verfahren eine Anzahl von Gruppen, welche dem Doppelten der Wurzel der der Anzahl von Peptiden nächst höheren Quadratzahl entspricht (insofern die Anzahl der Peptide nicht selbst eine Quadratzahl ist).

Als Beispiel sei das pp65-Protein des humanen Zytomegalievirus erwähnt. Es wurden 138 Peptide synthetisiert, welche die Aminosäuresequenz des gesamten Proteins (561 Aminosäuren) in voller Länge abdecken, wobei sich benachbarte Peptide um jeweils 9 Aminosäuren überlappen. 138 ist keine Quadratzahl. Die zu 138 nächste höhere Quadratzahl ist 144 (12x12). Es wurden die Peptide so in 2 x 12, also 24 Gruppen verteilt , dass jedes Peptid in genau zwei unterschiedliche Gruppen vorkommt. Durch die Kombination der Gruppen mit positiven Ergebnissen (Stimulation) lässt sich direkt auf das stimulierende Peptid schließen (bei nur zwei Gruppen mit positivem Ergebnis), oder aber auf eine kleine Anzahl von Kandidatenpeptiden, welche einzeln nachgetestet werden können, falls mehr als zwei Gruppen von Peptiden zu positiven Stimulationsergebnissen geführt haben. Das Prinzip dieser Anordnung ist ausführlich beschrieben bei F. Kern et al., Journal of Virology, Oktober 1999, p. 8179-8184. Eine Möglichkeit, *in einem einzigen Ansatz* mit entsprechender Negativkontrolle auszusagen, ob ein Protein auf CD8-T-Lymphozyten stimulierende Wirkung hat, ob also in der Aminosäuresequenz dieses Proteins von CD8-T-Lymphozyten erkannte Epitope vorliegen, ist bisher nicht beschrieben worden.

### Beschreibung der Erfindung

Es ist die Aufgabe der Erfindung, eine Möglichkeit anzubieten, wie Proteinantigene mit bekannter Sequenz zur Immunstimulation von CD8- und CD4-T-Lymphozyten eingesetzt werden können, wobei eine zelluläre Antigenprozessierung nicht notwendig ist und einzelne antigene Determinanten (Epitope) nicht identifiziert werden müssen. Es wurde nun gefunden, dass eine hinreichende Immunstimulierung durch Inkubation einer speziellen Peptidbibliothek aus Einzelfragmenten des Antigens mit einer gewissen Überlappung der Fragmente mit T-Lymphozyten erzielt werden kann. Die Stimulierung kann dabei durchflusszytometrisch erfasst werden. Somit kann festgestellt werden, ob ein Organismus (menschlicher oder tierischer Art) nach einer stattgehabten Exposition (auch gezielte Immunisierung) eine T-Lymphozyten-Antwort gegen das immunisierende Antigen aufgebaut hat. Diese T-Lymphozyten-Reaktivität kann im zeitlichen Verlauf untersucht werden. Es ist weiterhin Aufgabe der Erfindung ein Verfahren anzubieten, mit dem Proteinantigene, deren AminosäureSequenzen bekannt sind, in kurzer Zeit und mit vergleichsweise geringem Aufwand als T-Lymphozyten-stimulierende Proteinantigene identifiziert werden können. Damit ist weiterhin eine Möglichkeit gegeben, vor der Auswahl eines Proteins zur Identifizierung von Epitopen zu untersuchen, ob überhaupt T-Lymphozyten-stimulierende antigene Determinanten in diesem Protein vorliegen.

Die vorliegende Erfindung betrifft somit
(1) ein Verfahren zur antigen-spezifischen Stimulation von CD8- und/oder CD4-T-Lymphozyten mit synthetischen Peptidbibliotheken *in vitro,* das zum Nachweis einer T-Zell-Immunantwort oder zur Herstellung einer CD8- und/oder CD4-T-Lymphozyten-Zusammensetzung zur *in-vivo*-Behandlung von Mensch und Tier geeignet ist und die folgenden Schritte umfasst:
   (a) Unterteilen der Aminosäure-Sequenz des gesamten Proteins in Proteinfragmente mit Teil-Aminosäuresequenzen, wobei die Proteinfragmente eine Mindestlänge von 9 Aminosäureresten (AS) und eine Maximallänge von 25AS aufweisen und wobei angrenzende oder benachbarte Proteinfragmente sich mit ihrer Teil-Aminosäuresequenz überlappen,
   (b) Synthetisieren einer Peptidbibliothek, enthaltend die in (a) definierten Proteinfragmente und
   (c) Inkubieren einer Suspension mit den zu stimulierenden CD8- und/oder CD4-T-Lymphozyten mit allen Proteinfragmenten der Peptidbibliothek in einem einzigen Kulturansatz;
(2) in einer bevorzugten Ausführungsform des Verfahrens (1) ist dieses so ausgestaltet, dass es zur *in vitro* Immunstimulation von T-Lymphozyten von Säugern, insbesondere Menschen geeignet ist;
(3) stimulierte CD8- und CD4-T-Lymphozytenzusammensetzung, erhältlich gemäß dem Verfahren, wie vorstehend unter (2) definiert;
(4) ein Verfahren zum Identifizieren von stimulierenden oder nicht stimulierenden Mischungen aus allen Proteinfragmenten in einem einzigen Kulturansatz, wobei das Verfahren nach den Schritten (a) bis (c) wie vorstehend unter (1) definiert weiterhin folgende Schritte umfasst:
   d) Identifizieren, vorzugsweise durchflusszytometrisches Identifizierten, von
      (i) mindestens einem T-Zell-Zytokin, das durch das oder die Proteinfragmente induziert und in den T-Lymphozyten synthetisiert wurde und das intrazellulär oder an die Zellmembran gebunden vorliegt
         und/oder
      (ii) von mindestens einem Aktivierungsmarker, der durch das oder die Proteinfragmente induziert und in den T-Lymphozyten synthetisiert wurde und der intrazellulär oder an die Zellmembran gebunden vorliegt;
(5) die Verwendung der, wie vorstehend unter (4) identifizierten stimulierenden Mischungen, zur Herstellung eines Medikaments zur Stimulation von gegen das vorgenannte Gesamtprotein gerichtete CD8 und CD4-T-Lymphozyten zur antigen-spezifischen Immuntherapie bei Virusinfektionen und Allergien;
(6) Verwendung der durch das vorstehend unter (1) und (2) definierte Verfahren erhältlichen CD8- und CD4-T-Lymphozytenzusammensetzungen zur Herstellung eines Medikaments zur antigen-spezifischen Immuntherapie bei Virusinfektionen und Allergien, einschließlich der adoptiven Immuntherapie; und
(7) Zusammensetzung zur *in-vitro-* Immunstimulation von T-Lymphozyten von Säugern, umfassend die wie vorstehend unter (4) identifizierten stimulierenden Mischungen.

### Figurenbeschreibung

Figur 1: Peptide für Gesamtpool HCMV IE-1 (Laborstamm AD 169). Die Sequenz des Ausgangsproteins VIE1_HCMVA 55 KDA IMMEDIATE-EARLY PROTEIN 1 (IE1) - Human cytomegalovirus (strain AD169) ist aus Swiss-Prot P13202 bekannt und in SEQ ID NO:1 abgebildet.
Figur 2: Peptide für Gesamtpool HCMV pp65 (Laborstamm AD169). Die Sequenz des Ausgangsproteins PP65_HCMVA 65 KDA LOWER MATRIX PHOSPHOPROTEIN (PP65) - Human cytomegalovirus (strain AD169) ist aus Swiss-Prot P06725 bekannt und in SEQ ID NO:2 abgebildet.
Figur 3: Detektion von intrazellulär vorliegendem Interferon-γ in CD8⁺ T-Lymphozyten nach Stimulation mit den beschriebenen Peptidbibliotheken. Der Marker CD69 wurde neben Interferon-γ als Aktivierungsmarker verwendet. Die Darstellung ist auf CD3⁺/CD8⁺ Ereignisse eingegrenzt, angegeben ist die mittlere Fluoreszenzintensität.

### Detaillierte Beschreibung der Erfindung

"Antigene" in dem erfindungsgemäßen Verfahren sind solche Antigene, die eine Peptidgrundstruktur aufweisen (d. h. Proteine oder Polypeptide). Bei dem Antigen in Schritt (a) des vorstehend definierten Verfahrens handelt es sich um ein Antigen (d. h. Protein oder Polypeptid), gegen das eine T-Lymphozytenstimulation erwünscht ist bzw. bei dem getestet werden soll, ob eine solche Stimulation bereits erfolgte.

"Proteine oder Peptide" in der vorliegenden Erfindung haben als wesentliches Merkmal die Sequenz von mindestens neun AS.

Eine "Peptidbibliothek" im Sinne der Anmeldung ist eine komplexe Mischung aus Peptiden, welche in Ihrer Gesamtheit die komplette Sequenz eines Proteinantigens abdecken, und zwar so, dass sich entlang dieser Sequenz aufeinanderfolgende Peptide überlappen.

In dem Verfahren gemäß Ausführungsform (1) der Erfindung kann es daher notwendig sein - insbesondere dann, wenn die Aminosäuresequenz des Antigens nicht bekannt ist - vor dem vorstehend genannten Schritt (a) noch die Gesamtaminosäuresequenz des Antigens zu bestimmen.

Dabei ist gleichgültig, wie die Sequenz des Antigens ermittelt worden ist. So kann bei einem neuen Protein die Sequenz zum erstenmal analysiert werden oder bei bekannten Protein aus einer Datenbank abgelesen werden. Wichtig ist nur, dass die Aminosäuresequenz des Proteins oder Teilproteins bestimmt ist.

In einer bevorzugten Ausführungsform des erfindungsgemäßen Verfahrens (1) weisen die Proteinfragmente eine Mindestlänge von 15 AS auf. Es ist weiterhin bevorzugt, wenn zwischen benachbarten Proteinfragmenten eine Überlappung von 8 AS, vorzugsweise von 11 AS, vorliegt. Darüber hinaus können die synthetischen Proteinfragmente am N-Terminus und/oder C-Terminus je um maximal 7 natürliche oder künstliche AS und/oder eine Schutzgruppe verlängert sein. Bei diesen Verlängerungen aus natürlichen oder künstlichen AS handelt es sich um eine nicht überlappende Sequenz.

Geeignete Schutzgruppen am N-Terminus der Proteinfragmente sind dabei Alkyl-, Aryl-, Alkylaryl-, Aralkyl-, Alkylcarbonyl- oder Arylcarbonylgruppen mit 1 bis 10 Kohlenstoffatomen, eine Acylgruppe mit 1 bis 7 Kohlenstoffatomen usw. Bevorzugte Schutzgruppen für den N-Terminus sind die Naphthoyl-, Naphthylacetyl-, Naphthylpropionyl- und Benzoylgruppen. Geeignete Schutzgruppen für den C-Terminus der Proteinfragmente sind Alkoxy- oder Aryloxygruppen mit 1 bis 10 Kohlenstoffatomen oder aus eine Aminogruppe. Weitere Schutzgruppen sind in Houben-Weyl (1974) Georg Thieme Verlag, 4. Auflage beschrieben. Die Beschreibung der Schutzgruppen in der zitierten Literaturangabe ist Teil der Offenbarung.

Weiterhin ist bevorzugt, wenn die Konzentration der einzelnen Proteinfragmente der Peptidbibliothek wenigstens 1 ng/ml, vorzugsweise von ungefähr 0,1 bis ungefähr 10 µg/ml in dem Kulturansatz (Endkonzentration) beträgt. Besonders bevorzugt ist eine Konzentration von ungefähr 1 µg/ml Kulturansatz.

Darüber hinaus ist es bevorzugt, wenn die Inkubationslösung (d. h. der Kulturansatz) weiterhin eine oder mehrere Verbindungen mit kostimlatorischen Eigenschaften wie kostimulatorische Antikörper (z. B. anti-CD28 oder anti-CD49d) oder andere Moleküle mit kostimulatorischen Eigenschaften (z. B. stimulatorisches CTLA4-Ig) enthält. Diese Verbindungen sind vorzugsweise in Endkonznetrationen von 0,1 bis 10 µg/ml in dem Kulturansatz enthalten.

Eine besonders bevorzugte Ausführungsform des erfindungsgemäßen Verfahrens (1) zur antigen-spezifischen Stimulation von T-Lymphozyten mit synthetischen Peptidbibliotheken umfasst die folgenden Schritte:
(a₁) Ermitteln der Gesamt-Aminosäuresequenz des Antigens, welches ein Protein ist,
(a₂) Unterteilen der Gesamt-Aminosäure-Sequenz in Proteinfragmente mit Teil-Aminosäuresequenzen, wobei die Proteinfragmente eine Mindestlänge von 9 (bevorzugt 15) AS aufweisen, eine Maximallänge von 25 AS aufweisen und wobei angrenzende oder benachbarte Proteinfragmente sich mit Ihrer Teil-Aminosäuresequenz überlappen und eine Überlappung von 8 AS, besonders eine Überlappung von 11 AS, bevorzugt ist,
(b) Synthetisieren einer Peptidbibliothek, enthaltend die in (a₂) definierten Proteinfragmente, die gegebenenfalls am N-Terminus und/oder C-Terminus je um maximal 7 natürliche oder künstliche Aminosäuren und/oder eine Schutzgruppe verlängert sind,
(c) Inkubieren einer CD8- und/oder CD4-T-Lymphozyten enthaltenden Suspension mit allen Proteinfragmenten der Peptidbibliothek in einem einzigen Kulturansatz.
   Bevorzugt ist eine Verwendung des erfindungsgemäßen Verfahrens zum Identifizieren von stimulierenden oder nicht stimulierenden Mischungen aus allen Proteinfragmenten in einem einzigen Kulturansatz, wobei die folgenden Schritte hinzukommen:
d) Identifizieren (bevorzugt durchflusszytometrisches Identifizieren) von
   (i) mindestens einem T-Zell-Zytokin, das durch das oder die Proteinfragmente induziert und in den T-Lymphozyten synthetisiert wurde, dabei liegen das oder die Zytokine intrazellulär oder an die Zellmembran gebunden vor
      und/oder
   (ii) von mindestens einem Aktivierungsmarker, der durch das oder die Proteinfragmente induziert und in den T-Lymphozyten synthetisiert wurde, dabei liegen der oder die Aktivierungsmarker intrazellulär oder an die Zellmembran gebunden vor,

Das erfindungsgemäße Verfahren (1) ist auch geeignet festzustellen, ob T-Lymphozyten-stimulierende antigene Determinanten in einem

Antigen vorliegen.

Das erfindungsgemäße Verfahren ist weiterhin zur Diagnostik geeignet, insbesondere um festzustellen, ob ein Säuger, insbesondere ein Mensch, zuvor gegen ein spezifisches Protein mit seinem Immunsystem geantwortet hat und wie stark diese Antwort ist.

Gemäß der bevorzugten Ausführungsform (2) der Erfindung ist das Verfahren zur Immunstimulation von T-Lymphozyten von Säugern, insbesondere Menschen sowohl für *in-vitro*- als auch *in-vivo*-Applikationen geeignet. Dieses Verfahren kann weiterhin das Expandieren der stimulierten T-Lymphozyten umfassen.

Die vorstehend genannten Ausführungsformen des erfindungsgemäßen Verfahrens können auch so ausgestaltet sein, dass mehrere verschiedene synthetische Peptidbibliotheken (von unterschiedlichen Antigenen) gemeinsam in einem Kulturansatz oder in getrennten Kulturansätzen eingesetzt werden.

T-Lymphozyten enthaltende Suspensionen im Sinne dieser Anmeldung zeichnen sich dadurch aus, dass sie Zellen enthalten, welche MHC-gebundene Peptide präsentieren können. So können die präsentierenden Zellen neben den Antigen-präsentierenden Zellen auch zum Beispiel T-Lymphozyten sein.

Vorteilhaft bei dem erfindungsgemäßen Verfahren ist, dass das Identifizieren von mindestens einem T-Zell-Zytokin oder Aktivierungsmarkers auf der Einzelzell-Ebene erfolgt. Dadurch ist es möglich, den Phänotyp der reagierenden Zellen genau zu bestimmen. Zytokine und Oberflächenmarker sind ausführlich beschrieben in Abul K. ABBAS et al. (1997) Cellular and Molecular Immunology, Philadelphia, 3. Auflage, ISBN 0-7216-4024-9.

Es ist bekannt, dass an Moleküle der MHC-Klasse I (MHC = Major Histocompatibility Complex) bindende Proteinfragmente in der Regel eine Länge von 9 Aminosäuren aufweisen, während Proteinfragmente, welche an MHC-Klasse II Moleküle binden, etwas länger und in der Länge stärker variabel sind.

Vorteilhaft bei den erfindungsgemäßen Verfahren (1) und (2) ist, dass die Proteinfragmente trotz der kurzen Inkubationszeit von den MHC-Molekülen, die sich auf der Zelloberfläche befinden, ausreichend aufgenommen werden, um eine eindeutige Identifizierung einer T-Zell-Stimulation nach zum Beispiel sechs Stunden zu ermöglichen.

Bei dem erfindungsgemäßes Verfahren kann die T-Lymphozyten enthaltende Suspension aus Vollblut, peripheren weißen Blutzellen (PWBC), Milzzellen, Thymuszellen, Knochenmark, Liquor, aus Lymphknotenzellen usw. stammen.

In dem erfindungsgemäßen Verfahren ist besonders vorteilhaft, dass eine Aufarbeitung der T-Lymphozyten nicht erforderlich ist. So müssen die T-Lymphozyten nicht angereichert werden, weiterhin ist ein Entfernen oder Zerstören von anderen Zellen nicht notwendig. Hierdurch lässt sich das erfindungsgemäße Verfahren einfacher routinemäßig handhaben.

Bevorzugt ist ein erfindungsgemäßes Verfahren zur antigen-spezifischen Stimulation von T-Lymphozyten mit synthetischen Peptidbibliotheken, bei dem die T-Lymphozyten enthaltende Suspension aus Patienten, die therapiert werden sollen, aus anderen Spendern oder aus Tieren stammen. Stammt die T-Lymphozyten enthaltende Suspension aus einem Patienten, so lässt sich mit der Identifizierung zum Beispiel feststellen, gegen welches Protein eines Virus sich eine CD8- oder CD4-T-Lymphozyten-Antwort induzieren lässt. Die zur Untersuchung dieser Reaktivität eingesetzte Peptidbibliothek kann dann zur Stimulation weiterer T-Lymphozyten dieses oder anderer Patienten gezielt eingesetzt werden. Die so induzierten und zur Proliferation angeregten Zellen können *in vivo* oder *ex vivo* expandiert und anschließend dem Patienten retranfusioniert werden.

Das erfindungsgemäße Verfahren lässt sich auch in der Tiermedizin verwenden. Dabei sind unterschiedlichste Tierarten und auch Konstellationen von Tierpatienten und Spendern als Quelle der T-Lymphozyten enthaltenden Suspension denkbar.

Vorteilhaft ist ein erfindungsgemäßes Verfahren zur antigen-spezifischen Stimulation von T-Lymphozyten mit synthetischen Peptidbibliotheken , bei dem die Antigene, welche Proteine sind, aus Mikroorganismen, aus Makroorganismen, aus Zellen, Zellkulturen und/oder Geweben von Spendern oder Patienten stammen. Mikroorganismen sind zum Beispiel Viren, Bakterien, Pilze, Einzeller, Parasiten. Unter Makroorganismen fallen zum Beispiel alle mehrzelligen Eukaryoten. Gerade diese Quelle ist für die Beeinflussung von Allergien wichtig. Hierunter fallen Tiere und Pflanzen. Es können Zellen, Zellkulturen oder auch ganze Gewebe bestehend aus einer oder mehreren Schichten oder Zell-Typen verwendet werden.

Bevorzugt ist ein erfindungsgemäßes Verfahren zur antigen-spezifischen Stimulation von T-Lymphozyten mit synthetischen Peptidbibliotheken, wobei die Stimulation mittels eines Durchflusszytometers erfasst wird. Wesentlich ist dabei das Prinzip, dass Marker, die sich in der Zelle oder auf deren Oberfläche befinden, wie beispielsweise Zytokine oder Oberflächenmarker mit einem spezifischen Detektor, zum Beispiel einem Antikörper in Kontakt treten, wobei der Detektor mit einem Fluoreszenzfarbstoff beladen ist. Nach Anregung dieses Fluoreszenzfarbstoffes auf den in einem Flüssigkeitsstrom fokussierten Zellen durch Laserlicht zeichnet das Durchflusszytometer die emittierten Streulicht und Fluoreszenzsignale auf, was die zeitgleiche oder spätere Analyse der Zellen ermöglicht. Ausführlich sind solche Techniken beschreiben in Howard M. SHAPIRO (1995) Practical Flow Cytometry, New York, 3. Auflage, ISBN 0-471-30376-3. Die Detektion der intrazellulären Zytokine ist beschrieben in L. J. PICKER et al. (1995) Blood, Vol. 86, pp 1408.

Der Vorteil dieses erfindungsgemäßen Verfahrens zur antigen-spezifischen Stimulation von T-Lymphozyten mit synthetischen Peptidbibliotheken besteht darin, dass innerhalb von sehr kurzer Zeit und im Vergleich zur konventionellen Methode mit sehr geringem Aufwand ein Reagenz zur Immunstimulartion von T-Lymphozyten zur Verfügung gestellt werden kann. Dabei ist weiterhin vorteilhaft, dass einzelne stimulierende Epitope nicht identifiziert werden müssen.

In einem einzigen Ansatz (ein Tube oder 1 Well oder 1 Flask, etc) können die T-Lymphozyten eines Spenders/Patienten (CD8 und/oder CD4) gleichzeitig mit allen möglichen antigenen Determinanten des Proteins (bzw. der Proteine bei Verwendung mehrerer Peptidbibliotheken) stimuliert werden, ohne dass diese im Einzelnen bekannt sein müssen. Z.B. könnten die T-Lymphozyten eines Patienten nach Knochenmarktransplantation mit HLA-identischen dendritischen Zellen inkubiert werden, welche zuvor mt einem solchen Peptidgemisch inkubiert wurden, dabei könnten diese T-Lymphozyten mit allen für den gegebenen HLA-Typ relevanten (also bindenden) Epitopen stimuliert werden, ohne dass diese Epitope bekannt sein müssen oder durch das Verfahren bekannt werden. Entscheidend ist alleine, dass sie T-Lymphozyten stimulieren und zu dem oder den ausgewählten Protein(en) gehören. Diese Zellen könnten dem Patienten im Rahmen einer adoptiven Immuntherapie zurückgegeben werden.

Bevorzugt als Quelle der zu stimulierenden T-Lymphozyten sind solche (menschlichen oder tierischen) Spender, welche zuvor eine immunologische Primärantwort gegen das Antigen aufgebaut haben bzw. bei welchen eine solche Immunantwort gegen das Antigen durch Exposition induziert wurde. Dies kann beispielsweise im Rahmen einer Infektion stattgefunden haben oder auch im Rahmen einer Immunisierung. Auch bei einer Autoimmunantwort ist diese Situation gegeben.

Ein weiterer Vorteil besteht darin, dass der MHC-Typ des Spenders nicht bekannt sein muss. Ein weiterer Vorteil besteht darin, dass gleichzeitig und im gleichen Ansatz die Stimulation von CD8- und CD4-T-Lymphozyten untersucht werden kann.

Die stimulierten T-Lymphozyten gemäß Ausführungsform (3) der Erfindung werden vorzugsweise durch *in-vitro*-Stimulation erhalten. Die stimulierten Lymphozyten sind geeignet, einem Patienten transfusioniert zu werden.

Das Medikament gemäß Ausführungsform (5) der Erfindung kann neben üblichen Zusatz- und Hilfsstoffen noch weitere immunreaktive Verbindungen wie zum Beispiel die vorstehend definierten Verbindungen mit kostimulierenden Eigenschaften enthalten. Das Medikament kann auch mehrere der vorstehend definierten Peptidbibliotheken enthalten.

Die Zusammensetzung gemäß Ausführungsform (7) kann eine pharmazeutische Zusammensetzung sein, d. h. zur *in-vivo-*Behandlung von Mensch und Tier, oder eine diagnostische Zusammensetzung oder ein sogenannter Kit sein, d. h. primär zur *in-vitro*-Anwendung, wobei die Peptidbibliothek an das jeweils zu stimulierende Antigen angepasst ist. Hinsichtlich weiterer Komponenten der Zusammensetzung gilt das vostehend zu Ausführungsform (5) Ausgeführte.

Die vorliegende Erfindung wird anhand des nachfolgenden, nicht einschränkenden Beispiels näher erläutert.

### Beispiel

Mononukleare Zellen wurden aus dem durch venöse Punktion gewonnenen peripheren Blut zweier Patienten präpariert. Die Patienten besaßen Antikörper gegen das humane Zytomegalie-Virus (HCMV). Die nach Standardmethode präparierten Zellen wurden für sechs Stunden unter optimierten Bedingungen mit Peptidbibliotheken für die HCMV-Proteine 65 KD Lower Matrix Phosphoprotein (pp65) und 55 KD Immediate-Early Protein 1 (IE-1) inkubiert. Dies erfolgt gemäß dem in Kern et al., Eur. J. Immunol. 30:1676-1682 (2000) beschriebenen, die folgenden Schritte aufweisenden Verfahren:
1. Resuspendierung von PBMC (2,5 x 10 Exp6/ml in RPMI 1640 mit Zusatz von 2 mM Glutamin) nach Ficoll-Präparation (Standardvorschrift).
2. 400 µl dieser Suspension wurden in einem Inkubationsgefäß (steriles Röhrchen von Falcon Nr. 2054, 5 ml) mit 100 µl Peptidlösung (enthaltend 10 µg je Einzelpeptid in RPMI 1640 mit Zusatz von 2 mM Glutamin) versetzt.
3. Inkubation bei 37 °C und H₂O-gesättigter Atmosphäre mit 5 % CO₂ (Standardinkubator).
4. Nach 2 Stunden erfolgte die Zugabe von 500 µl RPMI 1640 mit Zusatz von 20 % fötalem Kälberserum (v/v) und zusätzlich Glutamin (2 mM) sowie 10 µg Brefeldin A (BFA, Endkonzentration im Ansatz 10 µg/ml). Die Endkonzentration von fötalem Kälberserum im Ansatz beträgt 10 % (v/v). Die Endkonzentration jedes einzelnen Peptides beträgt 1 µg/ml. BFA dient der Zurückhaltung synthetisierter Zytokine in den Zellen, was für die Detektion der intrazellulären Zytokine von Vorteil ist. Das Endvolumen des Ansatzes beträgt 1 ml.
5. Nach weiterer Inkubation über 4 h unter gleichen Bedingungen (also einer Gesamtinkubationsdauer von 6 h) erfolgte ein Inkubationsstop durch Zugabe von eiskalter PBS-Pufferlösung.
6. Es folgten eine Zentrifugation (8 min, 400 g), Dekantierung und die weitere Aufarbeitung der Proben nach Standardarbeitsvorschrift, inklusive Ablösung von der Röhrchenwand mittels 2 mM EDTA/PBS-Lösung, Fixierung, Permeabilisierung und Färbung mit monoklonalen Antikörpern.
7. Analyse am Durchflusszytometer (z. B. ein 4-Farben-Fluoreszenz-Durchflusszytometer vom Typ FacsCalibur (Becton Dickinson)).

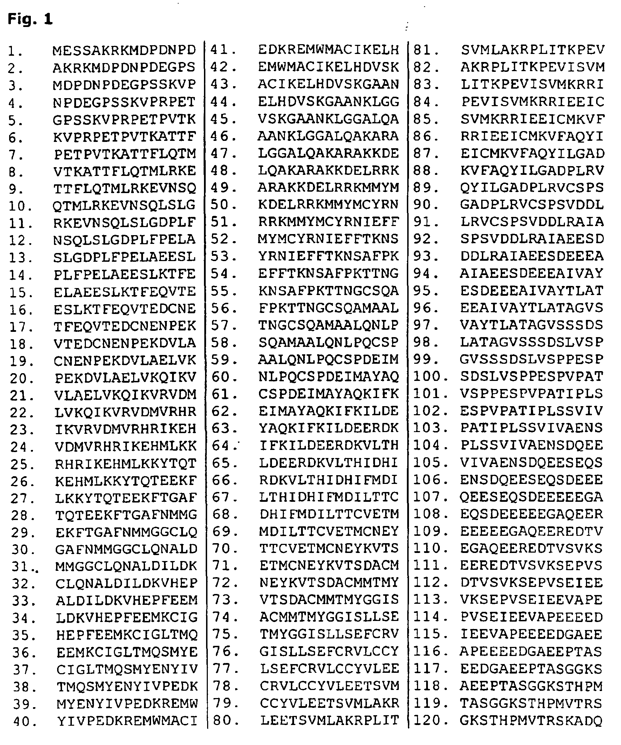

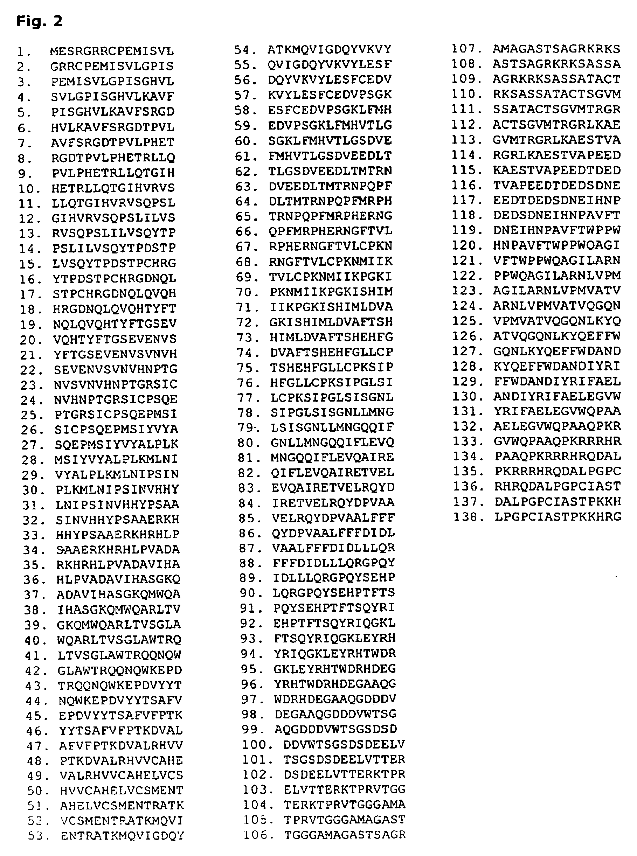

Die Sequenzen von IE-1 und pp65 sind in der SWISS-PROT Datenbank, European Bioinformatics Institute, unter den Nummern P13202 (s. auch SEQ ID NO:1) und P06725 (s. auch SEQ ID NO:2) abgelegt. Die Sequenzen beider Proteine sind darüber hinaus in M.S. Chee, A.T. Bankier, S. Becks et al., Curr. Top. Mircrobiol. Immunol. 154:125 - 169 (1990) beschrieben.
Dabei bestand die Peptidbibliothek, welche das 55KD Immediate-Early Protein 1 repräsentiert aus Peptiden mit 15 Aminosäuren Länge mit jeweils 9 Überlappungen zwischen aufeinanderfolgenden Peptiden (siehe Fig. 1), die Peptidbibliothek, welche das 65 KD Lower Matrix Phosphoprotein repräsentiert aus Peptiden mit 15 Aminosäuren Länge mit jeweils 11 Überlappungen zwischen aufeinanderfolgenden Peptiden (s. Fig. 2).

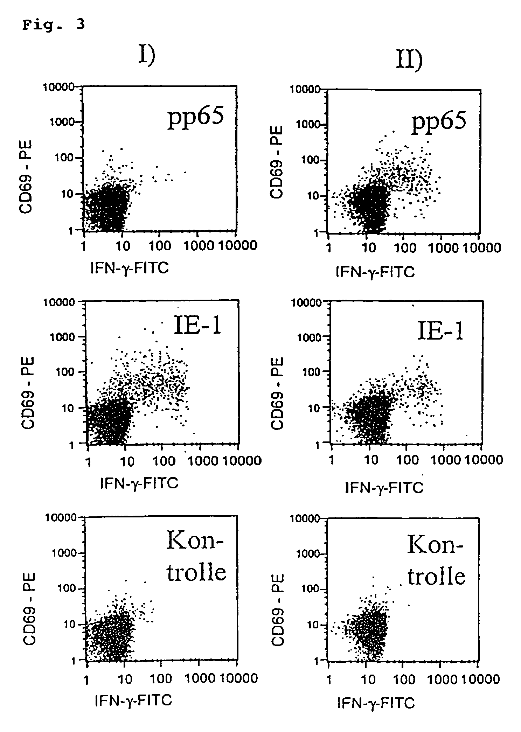

Die Inkubation mit den Peptidbibliotheken führte bei zwei verschiedenen Individuen (Spalten, "I)" und "II)" in Fig. 3) zur Produktion von IFN-y in T-Zellen, welches durch Messung am Durchflusszytometer auf Einzel-Zellebene (J. L. Picker et al., (1995) Blood, Vol 86, pp 1408-1419) nachgewiesen wurde oder aber zu keiner nachweisbaren Stimulation. Individuum I hatte eine CD8-T-Lymphozytenantwort gegen IE-1 aber nicht pp65, während Individuum II eine CD8-T-Lymphozytenantwort gegen beide Proteine hatte. Die Inkubation mit einem irrelevanten Peptid hatte diesen Effekt nicht (Kontrolle).

## Patentansprüche

1. Verfahren zur antigen-spezifischen Stimulation von CD8- und/oder CD4-T-Lymphozyten mit synthetischen Peptidbibliotheken *in vitro,* das zum Nachweis einer T-Zell-Immunantwort oder zur Herstellung einer CD8- und/oder CD4-T-Lymphozyten-Zusammensetzung zur *in-vivo*-Behandlung von Mensch und Tier geeignet ist und die folgenden Schritte umfasst:
(a) Unterteilen der Aminosäure-Sequenz des gesamten Proteins in Proteinfragmente mit Teil-Aminosäuresequenzen, wobei die Proteinfragmente eine Mindestlänge von 9 Aminosäureresten (AS) und eine Maximallänge von 25AS aufweisen und wobei angrenzende oder benachbarte Proteinfragmente sich mit ihrer Teil-Aminosäuresequenz überlappen,
(b) Synthetisieren einer Peptidbibliothek, enthaltend die in (a) definierten Proteinfragmente und
(c) Inkubieren einer Suspension mit den zu stimulierenden CD8- und/oder CD4-T-Lymphozyten mit allen Proteinfragmenten der Peptidbibliothek in einem einzigen Kulturansatz.

2. Verfahren nach Anspruch 1, wobei zwischen benachbarten Proteinfragmenten eine Überlappung von 8 AS, vorzugsweise von 11 AS, vorliegt.

3. Verfahren nach einem oder mehreren der Ansprüche 1 und 2, wobei die synthetischen Proteinfragmente am N-Terminus und/oder C-Terminus je um maximal 7 natürliche oder künstliche AS und/oder eine Schutzgruppe verlängert sind.

4. Verfahren nach einem oder mehreren der Ansprüche 1 bis 3, wobei die Konzentration der einzelnen Proteinfragmente der Peptidbibliothek wenigstens 1 ng/ml, vorzugsweise von 0,1 bis 10 µg/ml in dem Kulturansatz beträgt.

5. Verfahren nach einem oder mehreren der Ansprüche 1 bis 4, wobei der Inkubationslösung eine oder mehrere Verbindungen mit kostimulatorischen Eigenschaften, ausgewählt aus den kostimulatorischen Antikörpern, wie Anti-CD28 und AntiCD49d, und CTLA4-Ig zugegeben werden.

6. Verfahren nach einem oder mehreren der Ansprüche 1 bis 5, wobei in dem Verfahren vor Schritt (a) die Gesamt-Aminosäurensequenz des gesamten Proteins bestimmt wird.

7. Verfahren zum Identifizieren von stimulierenden oder nicht stimulierenden Mischungen aus allen Proteinfragmenten in einem einzigen Kulturansatz, wobei das Verfahren nach den Schritten (a) bis (c) von Anspruch 1 weiterhin folgende Schritte umfasst:
d) Identifizieren, vorzugsweise durchflusszytometrisches Identifizieren, von
(i) mindestens einem T-Zell-Zytokin, das durch das oder die Proteinfragmente induziert und in den T-Lymphozyten synthetisiert wurde und das intrazellulär oder an die Zellmembran gebunden vorliegt
und/oder
(ii) von mindestens einem Aktivierungsmarker, der durch das oder die Proteinfragmente induziert und in den T-Lymphozyten synthetisiert wurde und der intrazellulär oder an die Zellmembran gebunden vorliegt.

8. Verfahren nach einem oder mehreren der Ansprüche 1 bis 4, das geeignet ist festzustellen, ob T-Lymphozyten-stimulierende antigene Determinanten in einem Antigen vorliegen.

9. Verfahren nach einem oder mehreren der Ansprüche 1 bis 6, das zur *in vitro* Immunstimulation von T-Lymphozyten von Säugern, insbesondere von Menschen geeignet ist.

10. Verfahren nach Anspruch 9, weiterhin umfassend Expandieren der stimulierten T-Lymphozyten.

11. Verfahren nach einem oder mehreren der Ansprüche 1 bis 6, das zum Nachweis einer T-Zell-Immunantwort, nämlich zum Feststellen, ob ein Säuger, insbesondere ein Mensch, zuvor gegen wenigstens ein Proteinfragment des gesamten Proteins von Schritt (a) von Anspruch 1 mit seinem Immunsystem geantwortet hat und wie stark diese Antwort ist, geeignet ist.

12. Verfahren nach einem oder mehreren der Ansprüche 8 bis 11, das die Verwendungen von mehreren verschiedenen synthetischen Peptidbibliotheken umfasst, wobei die Inkubation der Peptidbibliotheken mit der CD8- und/oder CD4-T-Lymphozyten-Suspension gemeinsam in einem Kulturansatz oder in getrennten Kulturansätzen erfolgt.

13. Stimulierte CD8- und CD4-T-Lymphozytenzusammensetzung, erhältlich gemäß dem Verfahren nach Anspruch 9 oder 10.

14. Stimulierte CD8- und CD4-T-Lymphozytenzusammensetzung nach Anspruch 13, die zum Transfusionieren an einem Patienten geeignet ist.

15. Verwendung der in Anspruch 7 identifizierten stimulierenden Mischungen, enthaltend die Proteinfragmente mit Teil-AS-Sequenzen der Gesamt-AS-Sequenz des Gesamtproteins, wobei die Proteinfragmente eine Mindestlänge von 9 Aminosäureresten (AS) und eine Maximallänge von 25AS aufweisen und wobei angrenzende oder benachbarte Proteinfragmente sich mit ihrer Teil-Aminosäuresequenz überlappen, zur Herstellung eines Medikaments zur Stimulation von gegen das vorgenannte Gesamtprotein gerichtete CD8- und CD4-T-Lymphozyten zur antigen-spezifischen Immuntherapie bei Virusinfektionen und Allergien.

16. Verwendung der durch das Verfahren gemäß einem oder mehreren der Ansprüche 1 bis 6, 8 und 9 erhältlichen CD8- und CD4-T-Lymphozytenzusammensetzung zur Herstellung eines Medikaments zur antigen-spezifischen Immuntherapie bei Virusinfektionen und Allergien, einschließlich der adoptiven Immuntherapie.

17. Zusammensetzung zur *in-vitro*-Immunstimulation von T-Lymphozyten von Säugern, umfassend die in Anspruch 7 identifizierten stimulierenden Mischungen, enthaltend die Proteinfragmente mit Teil-AS-Sequenzen der Gesamt-AS-Sequenz des Gesamtproteins, wobei die Proteinfragmente eine Mindestlänge von 9 Aminosäureresten (AS) und eine Maximallänge von 25AS aufweisen und wobei angrenzende oder benachbarte Proteinfragmente sich mit ihrer Teil-Aminosäuresequenz überlappen.

## Claims

1. A method for the antigen-specific stimulation of CD8+ and/or CD4+ T lymphocytes with synthetic peptide libraries *in vitro* that is suitable for detecting a T cell immune response or for preparing a CD8+ and/or CD4+ T lymphocyte composition for the *in vivo* treatment of humans and animals, comprising the following steps:
(a) subdividing the amino acid sequence of the total protein into protein fragments with partial amino acid sequences, wherein said protein fragments have a minimum length of 9 amino acid residues (AAs) and a maximum length of 25 AAs, and wherein adjacent or neighboring protein fragments are overlapping with their partial amino acid sequence;
(b) synthesizing a peptide library containing the protein fragments defined in (a); and
(c) incubating a suspension containing the CD8+ and/or CD4+ T lymphocytes to be stimulated with all the protein fragments of said peptide library in a single culture run.

2. The method according to claim 1, wherein an overlap of 8 AAs, preferably 11 AAs, exists between neighboring protein fragments.

3. The method according to one or more of claims 1 and 2, wherein the synthetic protein fragments are extended by a maximum of 7 natural or artificial AAs and/or a protective group at the N terminus and/or C terminus.

4. The method according to one or more of claims 1 to 3, wherein the concentration of the individual protein fragments of the peptide library is at least 1 ng/ml, preferably from 0.1 to 10 µg/ml, in the culture mix.

5. The method according to one or more of claims 1 to 4, wherein one or more compounds having costimulatory properties and selected from the costimulatory antibodies, such as anti-CD28 and anti-CD49d, and CTLA4-Ig are added to the incubation solution.

6. The method according to one or more of claims 1 to 5, wherein the total amino acid sequence of the total protein is determined prior to step (a) in the method.

7. A method for identifying stimulating or non-stimulating mixtures of all protein fragments in a single culture run, wherein the method of claim 1 further comprises the following steps after steps (a) to (c): (d) identifying, preferably flow-cytometric identifying, of
(i) at least one T-cell cytokine which was induced by the protein fragment or fragments and synthesized in the T lymphocytes and which is intracellular or bound to the cell membrane;
and/or
(ii) at least one activation marker which was induced by the protein fragment or fragments and synthesized in the T lymphocytes and which is intracellular or bound to the cell membrane.

8. The method according to one or more of claims 1 to 4 which is capable of establishing whether T-lymphocyte-stimulating antigenic determinants are present in an antigen.

9. The method according to one or more of claims 1 to 6 which is adapted for *in vitro* immunostimulation of T lymphocytes of mammals, especially humans.

10. The method according to claim 9, further comprising expansion of the stimulated T lymphocytes.

11. The method according to one or more of claims 1 to 6 which is suitable for detecting a T cell immune response, namely to establish whether a mammal, especially a human, has previously responded to at least one protein fragment of the whole protein of step (a) with its immune system, and if so, how strong such response is.

12. The method according to one or more of claims 8 to 11 which comprises the use of several different synthetic peptide libraries, wherein the incubation of the peptide libraries with the CD8+ and/or CD4+ T lymphocyte suspension is effected together in one culture run or in separated culture runs.

13. A stimulated CD8+ and CD4+ T lymphocyte composition obtainable by the method according to claim 9 or 10.

14. The stimulated CD8+ and CD4+ T lymphocyte composition according to claim 13, which is capable of being transfused into a patient.

15. Use of the stimulating mixtures identified in claim 7 and containing the protein fragments with partial AA sequences of the total AA sequence of the total protein, wherein said protein fragments have a minimum length of 9 amino acid (AA) residues and a maximum length of 25 AAs, and wherein adjacent or neighboring protein fragments are overlapping with their partial amino acid sequence, for the preparation of a medicament for stimulating CD8+ and CD4+ T lymphocytes directed against the above mentioned total protein for antigen-specific immunotherapy in virus infections and allergies.

16. Use of the CD8+ and CD4+ T lymphocyte composition obtainable by the method according to one or more of claims 1 to 6, 8 and 9 for the preparation of a medicament for antigen-specific immunotherapy in virus infections and allergies including adoptive immunotherapy.

17. A composition for the *in vitro* immunostimulation of T lymphocytes of mammals, comprising the stimulating mixtures identified in claim 7 and containing the protein fragments with partial AA sequences of the total AA sequence of the total protein, wherein said protein fragments have a minimum length of 9 amino acid (AA) residues and a maximum length of 25 AAs, and wherein adjacent or neighboring protein fragments are overlapping with their partial amino acid sequence.

## Revendications

1. Procédé de stimulation spécifique de l'antigène des lymphocytes T CD8 et/ou CD4 avec des banques de peptides de synthèse *in vitro,* qui est approprié pour détecter une réponse immunitaire des cellules T ou pour fabriquer une composition de lymphocytes T CD8 et/ou CD4 destinée au traitement *in vivo* des hommes et des animaux, et qui comprend les étapes suivantes :
(a) subdivision de la séquence d'acides aminés de la protéine totale en fragments de protéine comportant des séquences partielles d'acides aminés, où les fragments de protéine présentent une longueur minimum de 9 résidus acide aminé (AA) et une longueur maximum de 25 AA, et où les fragments de protéines contigus ou voisins se chevauchent par leur séquence partielle d'acides aminés,
(b) synthétisation d'une banque de peptides contenant les fragments de protéine définis en (a) et
(c) incubation d'une suspension comportant les lymphocytes T CD8 et/ou CD4 à stimuler avec tous les fragments de protéine de la banque de peptides dans une préparation de culture unique.

2. Procédé selon la revendication 1, dans lequel il existe entre les fragments de protéine voisins un chevauchement de 8 AA, de préférence de 11 AA.

3. Procédé selon une ou plusieurs des revendications 1 et 2, dans lequel les fragments de protéine de synthèse sont prolongés en extrémité N et/ou en extrémité C respectivement d'un maximum de 7 AA naturels ou synthétiques et/ou d'un groupe protecteur.

4. Procédé selon une ou plusieurs des revendications 1 à 3, dans lequel la concentration des différents fragments de protéine de la banque de peptides est d'au moins 1 ng/ml, de préférence de 0,1 à 10 µg/ml dans la préparation de culture.

5. Procédé selon une ou plusieurs des revendications 1 à 4, dans lequel on ajoute à la solution d'incubation un ou plusieurs composés présentant des propriétés co-stimulantes, sélectionnés parmi les anticorps co-stimulants, tels que des anti-CD28 et des anti-CD49d, et des CTLA4-Ig.

6. Procédé selon une ou plusieurs des revendications 1 à 5, dans lequel, dans le procédé, on détermine avant l'étape (a) la séquence totale d'acides aminés de la protéine entière.

7. Procédé d'identification de mélanges stimulants ou non stimulants composés de tous les fragments de protéine dans une préparation de culture unique, dans lequel le procédé, après les étapes (a) à (c) de la revendication 1, comprend en outre les étapes suivantes :
d) identification, de préférence identification par cytométrie de flux, de
(i) au moins une cytokine des cellules T qui a été induite par le ou les fragments de protéine et a été synthétisée dans les lymphocytes T et qui se présente de façon intracellulaire ou liée à la membrane de la cellule, et/ou
(ii) au moins un marqueur d'activation qui a été induit par le ou les fragments de protéine et a été synthétisé dans les lymphocytes T et qui se présente de façon intracellulaire ou lié à la membrane de la cellule.

8. Procédé selon une ou plusieurs des revendications 1 à 4, qui est approprié pour déterminer si des déterminants antigènes stimulant les lymphocytes T existent dans un antigène.

9. Procédé selon une ou plusieurs des revendications 1 à 6, qui est approprié pour la stimulation immunitaire *in vitro* des lymphocytes T de mammifères, en particulier des hommes.

10. Procédé selon la revendication 9, comprenant en outre l'expansion des lymphocytes T stimulés.

11. Procédé selon une ou plusieurs des revendications 1 à 6, qui est approprié pour détecter une réponse immunitaire des cellules T, à savoir pour déterminer si un mammifère, en particulier un homme, a précédemment répondu avec son système immunitaire contre au moins un fragment de protéine de la protéine totale de l'étape (a) de la revendication 1, et quelle est l'intensité de cette réponse.

12. Procédé selon une ou plusieurs des revendications 8 à 11, qui comprend les utilisations de plusieurs banques de peptides de synthèse différentes, dans lequel l'incubation des banques de peptides avec la suspension de lymphocytes T CD8 et/ou CD4 se fait conjointement dans une seule préparation de culture ou dans des préparations de culture séparées.

13. Composition de lymphocytes T CD8 et CD4 stimulés pouvant être obtenue selon le procédé selon la revendication 9 ou 10.

14. Composition de lymphocytes T CD8 et CD4 stimulés selon la revendication 13, qui est appropriée pour transfuser un patient.

15. Utilisation des mélanges stimulants identifiés dans la revendication 7, contenant les fragments de protéine ayant des séquences partielles d'AA de la séquence totale d'AA de la protéine entière, dans laquelle des fragments de protéine présentent une longueur minimum de 9 résidus acide aminé (AA) et une longueur maximum de 25 AA et dans laquelle les fragments de protéines contigus ou voisins se chevauchent par leur séquence partielle d'acides aminés, pour fabriquer un médicament destiné à la stimulation des lymphocytes T CD8 et CD4 dirigée contre la protéine entière précitée, à des fins d'immunothérapie spécifique de l'antigène en cas d'infections virales et d'allergies.

16. Utilisation de la composition de lymphocytes T CD8 et CD4 pouvant être obtenue selon une ou plusieurs des revendications 1 à 6, 8 et 9 pour fabriquer un médicament destiné à une immunothérapie spécifique de l'antigène en cas d'infections virales et d'allergies, notamment l'immunothérapie adoptive.

17. Composition destinée à la stimulation immunitaire in vitro des lymphocytes T de mammifères, comprenant les mélanges stimulants identifiés dans la revendication 7, contenant les fragments de protéine ayant les séquences partielles d'AA de la séquence totale d'AA de la protéine entière, dans laquelle les fragments de protéine présentent une longueur minimum de 9 résidus acide aminé (AA) et une longueur maximum de 25 AA et dans laquelle les fragments de protéines contigus ou voisins se chevauchent par leur séquence partielle d'acides aminés.
